# EUROPEAN PATENT SPECIFICATION

(11) **EP 0 664 287 B1**
(45) Date of publication and mention of the grant of the patent: **15.07.1998**
(21) Application number: 95200063.6
(22) Date of filing: 12.01.1995
(51) Int. Cl.: C07C 401/00, A61K 31/59

(54) **Vitamin D compounds and method of preparing these compounds**
Vitamin-D-Derivate und Verfahren zu deren Herstellung
Dérivés de vitamine D et leur procédé de préparation

(30) Priority: 20.01.1994 EP 94200131
(43) Date of publication of application: 26.07.1995
(73) Proprietor: DUPHAR INTERNATIONAL RESEARCH B.V, NL-1381 CP Weesp (NL)
(72) Inventor: Torneiro, Mercedes, NL-1380 AC Weesp (NL); Fall, Yagamare, NL-1380 AC Weesp (NL); Mourino, Antonio, NL-1380 AC Weesp (NL); Zorgdrager, Jan, NL-1380 AC Weesp (NL); van de Velde, Jan-Paul, NL-1380 AC Weesp (NL)
(74) Representative: Swaters, Pieter D.

(56) References cited:
- EP-A- 0 387 077
- WO-A-85/02189
- WO-A-85/03298
- THE JOURNAL OF ORGANIC CHEMISTRY, vol.51, no.8, 18 April 1986, WASHINGTON DC, US pages 1264 - 1269 F. J. SARDINA ET AL 'Studies on the synthesis of side-chain hydroxylated metabolites of vitamin D. 2. Stereocontrolled synthesis of 25-hydroxyvitamin D2.'
- TETRAHEDRON LETTERS, vol.28, no.39, 1987, OXFORD, GB pages 4589 - 4590 L. CASTEDO ET AL 'Stereoselective synthesis of 25-hydroxyvitamin D2 side chain via the acetal template route.'

## Description

The invention relates to new vitamin D compounds, to a method of preparing these compounds and to their use in pharmacology. The invention further relates to valuable new intermediates.

It is generally known, that vitamin D compounds or vitamin D related compounds ("vitamin D compounds") have a strong biological activity and may be used in all those cases in which problems with the calcium metabolism and bone metabolism play a part. A few years ago it was found that various active vitamin D compounds also have other pharmacotherapeutic activities and may be used successfully, for example, for the treatment of certain skin diseases, for cosmetic applications and for treating diseases which are related to cell differentiation, cell proliferation or imbalance in the immune system, including diabetes mellitus, hypertension and inflammatory diseases such as rheumatoid arthritis and asthma. In addition, these compounds may be used in various veterinary applications, and for diagnostic purposes.

It is therefore of the utmost importance to have the disposal of an arsenal of active vitamin D compounds for the above various application fields so as to be able to make the best possible choice of a vitamin D compound for the application in view.

Vitamin D compounds which are of interest for the above applications are hydroxylated vitamin D compounds, in particular vitamin D compounds hydroxylated in the 1α-, 24- and/or 25-positions. Recent developments in the field of active vitamin D compounds are 19-nor-vitamin D compounds (EP-A-0387077), 25,25-di(cyclo)alkyl vitamin D compounds (non-prepublished US patent application 08/070,998) and (C-18)-modified vitamin D compounds (EP-A-0521550), preferably also hydroxylated in the 1α-position and optionally in the (C-17)-side chain. Other modifications of the (C17)-side chain have been proposed, likewise to improve the intended activity and to suppress detrimental side-effects. Examples of modifications of the (C17)-side chain are chain elongations (homo compounds), 22-oxa modifications, fluor substitutions, epoxy groups (e.g. WO 92/21695), etc. Generally, however, the above (C17)-side chain modified vitamin D compounds are still not completely satisfactory as regards their selective activity, i.e. the intended activity without detrimental side-effects, such as high calcemic and calciuric effects.

It is well-known in the art, that vitamin D₃ metabolizes in the living organism into a number of hydroxylated vitamin D₃ compounds, viz. 25-hydroxy-vitamin D₃ and 1α,25-dihydroxy-vitamin D₃. The metabolism of vitamin D₂ is thought to be related to that of vitamin D₃, also resulting in a number of hydroxylated vitamin D₂ metabolites. To investigate these hydroxylated vitamin D₂ metabolites, Sardina et al. (J. Org. Chem., Vol. 51, 1986, 1264-1269) have synthetized 25-hydroxyvitamin D₂, Granja et al. (J. Org. Chem., Vol. 58, 1993, 124-131) have recently synthetized 1α,25-dihydroxy-vitamin D₂, and Choudhry et al. (J. Org. Chem., Vol. 58, 1993, 1496-1500) have - also recently - succeeded in the synthesis of 1α,25,28-trihydroxy-vitamin D₂. Although during the last decade a great number of publications has been devoted to vitamin D₃ analogues and derivatives as well as to their biological activity, vitamin D₂ compounds have not been explored largely, apparently (also) due to their difficult synthetic accessibility.

It is therefore the objective of the present invention to provide a new class of vitamin D compounds, in particular vitamin D₂ compounds and related compounds, which is well accessible from readily available or accessible starting materials.

According to the present invention this objective can be achieved with a new vitamin D compound of the general formula 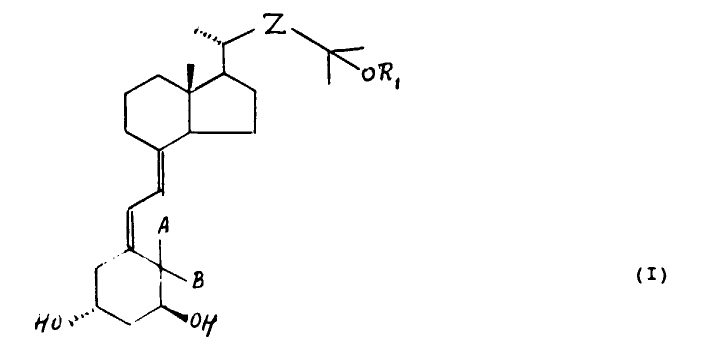 wherein:
R₁ is a hydrogen atom or a hydroxy protecting group;
Z is a group of the general formula 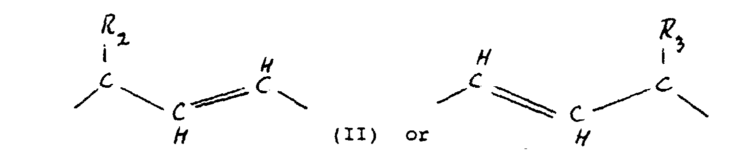 wherein
   R₂ and R₃ are each individually straight or branched (C₁-C₆)alkyl groups or (C₂-C₆)alkenyl groups,
      optionally substituted with one or more substituents selected from hydroxy, (C₁-C₄)alkoxy, fluoro, (C₃-C₆)cycloalkyl and (C₂-C₃)alkylenedioxy; or with unsubstituted or substituted phenyl;
   or (C₃-C₆)cycloalkyl groups, or unsubstituted or substituted phenyl groups;
   or wherein R₁ and R₃ together with the interconnecting oxadimethylene biradical form a 5-7 membered ring-closed (C₁-C₄)alkyl substituted hemiacetal;
   with the proviso, that R₂ and R₃ are not methyl groups;
A and B are each individually hydrogen atoms or methyl groups, or
A and B form together a methylene group.

The above new vitamin D compounds of the invention, presented by the general formula I, are valuable substances. The biological results indicate that these compounds are promising as biologically active substances and may be used in all above-mentioned pharmacotherapeutic indications, more in particular for the treatment of osteoporosis, renal osteodystrophy, osteomalacia, skin disorders such as psoriasis (and other hyperproliferative skin diseases), eczema and dermatitis, myopathy, leukemia, breast and colon cancer, osteosarcomas, squamous cell carcinomas, melanoma, certain immunological disorders, and transplant rejections.
Furthermore, the new vitamin D compounds of the invention may be used for wound healing and may be incorporated in cosmetic compositions, such as creams, lotions, ointments and the like, in order to preserve, condition and/or protect the skin and to improve various skin conditions, such as wrinkles, dry skin, skin slackness and insufficient sebum secretion. The new vitamin D compounds may also be used for diagnostic purposes.

Suitable substituents of the above phenyl group are: (C₁-C₄)alkyl, (C₁-C₄)alkoxy, fluoro, trifluoromethyl and hydroxy.

Preferred is a vitamin D compound of the general formula 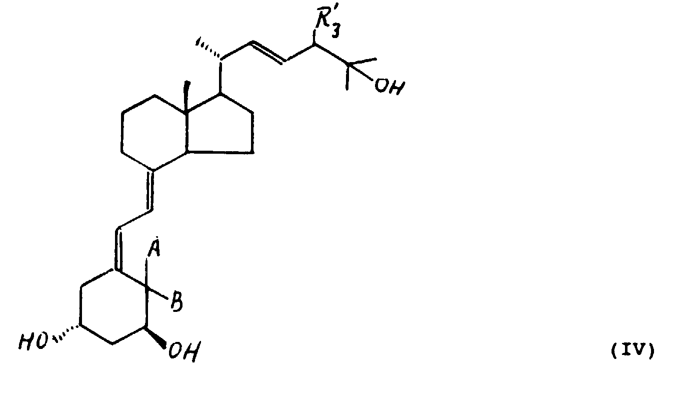 wherein:
A and B have the meanings given above, and
R₃' is a straight or branched (C₂-C₆)alkyl group, optionally substituted with hydroxy, (C₁-C₂)alkoxy or (C₂-C₃)alkylenedioxy, a cyclopropyl group or a phenyl group.

It is a merit of the present invention, that the desired C-24 stereo-isomer can easily be obtained, as will be explained hereinafter. Therefore the present invention also relates to a vitamin D compound of the above general formula IV, wherein the C-24 atom, i.e. the carbon atom to which R₁' is attached, has either the **R** or the **S** configuration.

The invention also relates to a method of preparing a vitamin D compound of the above formula I as defined above, in that a hydrindane compound of the general formula 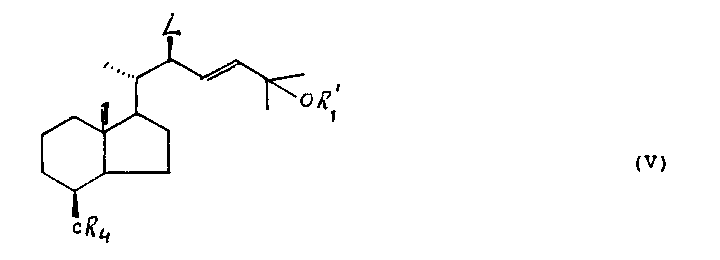 wherein:
L is a suitable leaving group, and
R₁' and R₄ are each individually hydroxy-protecting groups; is reacted with a reagent of the general formula

   R₃-MgX or R₃-Li

   wherein
   R₃ has the meaning given hereinbefore, and
   X is a halogen atom,
in the presence of a Cu(I) compound;
after which the hydrindane intermediate obtained, having the general formula 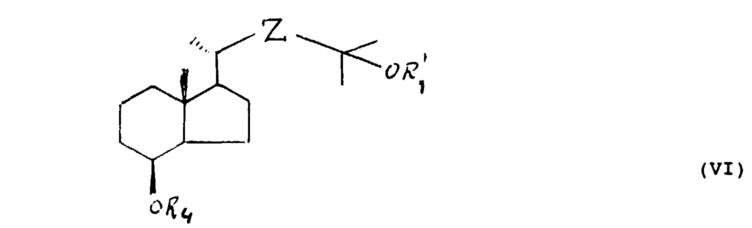 wherein Z has the meaning given above,
is oxidized, after deprotection of OR₄, to the corresponding hydrindane-4-one compound, and is then converted, in a manner known per se for related compounds,
either (a) with a Wittig reagent of the general formula 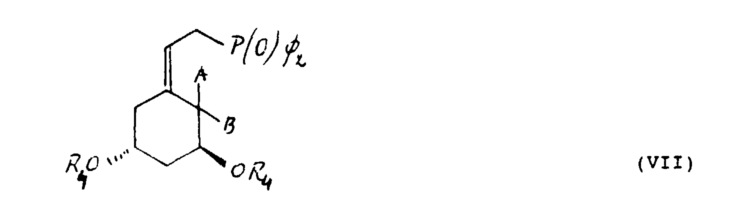 wherein:
R₄ has the above meaning, and
A and B have the meanings given hereinbefore,
or (b), after enolization, with an enyne compound of the general formula 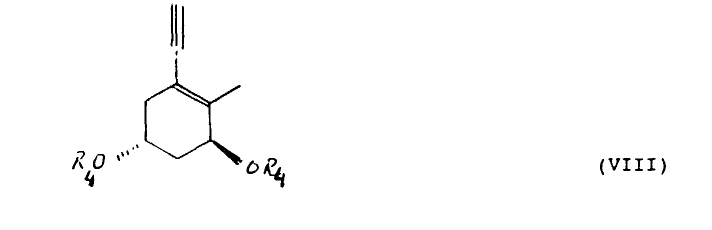 wherein R₄ has also the above meaning, followed by hydrogenation and isomerization, to produce a compound of the general formula I, wherein A and B form together a methylene group;
followed by deprotection.

Alternatively, the vitamin compound of the above formula I as defined above can be prepared with a method wherein a hydrindane compound of the general formula V as defined above is oxidized, after deprotection of OR₄, to the corresponding hydrindane-4-one compound, and is then converted, in a manner known per se for related compounds,
either (a) with a Wittig reagent of the general formula VII as defined above to produce a compound of the general formula 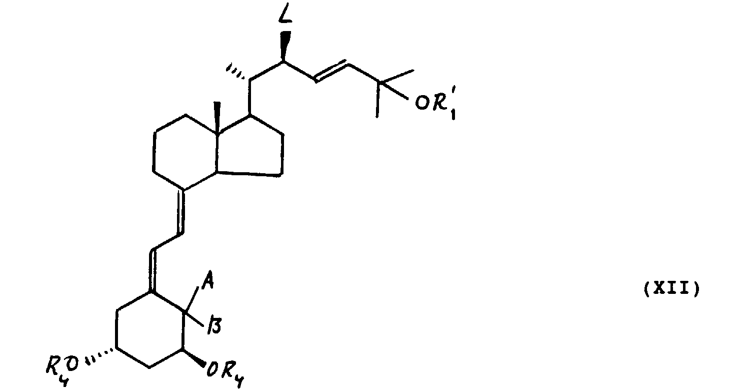 wherein:
L is a suitable leaving group;
R₁' and R₄ are each individually hydroxy protecting groups; and
A and B are each individually hydrogen atoms or methyl groups,
or A and B form together a methylene group
or (b) after enolization with an enyne compound of the general formula VIII as defined above, followed by hydrogenation and isomerization, to produce a compound of the general formula XII, wherein A and B form together a methylene group;
followed by reaction with a reagent of the general formula

R₃-MgX or R₃-Li

wherein:
R₃ has the meaning given hereinbefore, and
X is a halogen atom,
in the presence of a Cu(I) compound;
followed by deprotection.

Examples of suitable leaving groups L are: phosphate esters, sulphonates such as tosylate and mesylate, and N-arylcarbamates.

Hydroxy groups in the above intermediates or reactants may be protected by a reaction with a suitable etherification agent; for example, a methoxymethylating agent (such as methoxymethylchloride), a trialkylsilylimidazole, a trialkylsilylhalide, a trialkyl-silyltriflate (-trifluoromethanesulfonate), a diphenylalkylsilylhalide, or a diphenylalkylsilyltriflate, or a derivative thereof, the alkyl groups of which have 1 to 6 carbon atoms. Particularly suitable for this purpose are trimethylsilylchloride, tert.-butyldimethylsilylchloride, dimethyl-(1,1,2-trimethylpropyl)-silylchloride, tert.-butyldimethylsilyl triflate, or trimethylsilyl-imidazole, because these etherification agents readily react with the hydroxy group to be protected to form an ether function, which on the one hand is sufficiently stable under the conditions of the reaction or reactions in view, but on the other hand can easily be removed [deprotection] to recover the original hydroxy group; tert.-butyldimethylsilylchloride or triflate is to be preferred, because the tert.-butyldimethylsilyl group has been found to be excellently suitable as a protective group.
The enolic hydroxy group is preferably derivatized by a reaction with N-phenyltriflimide to produce a triflate.

The starting compounds of formula V can conveniently be prepared from readily available substances, e.g. as follows:

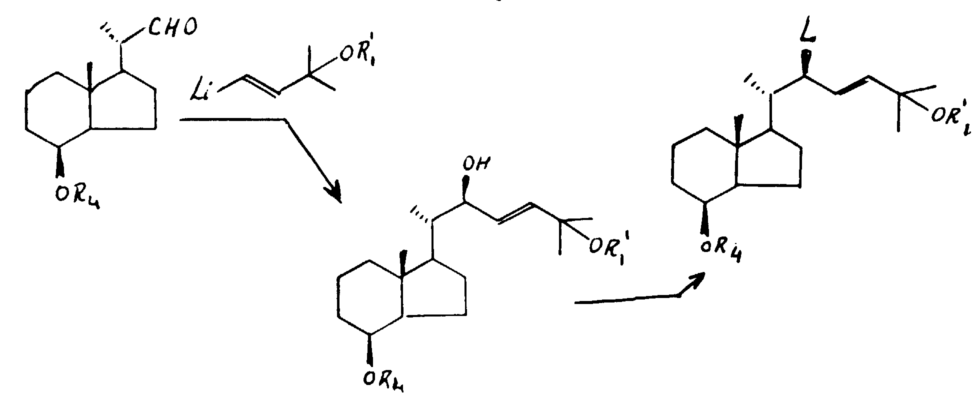

The second reaction step, i.e. the conversion of the hydroxy group in the intermediate allylic hydroxy compound to a leaving group L can be performed with the aid of various compounds, viz.:
- sulphonating compounds, such as tosylchlorde and mesylchloride;
- phosphate-introducing compounds, such as ClPO(O-hydrocarbyl)₂; and
- N-arylcarbamoyl-introducing compounds, such as phenylisocyanate.
The term hydrocarbyl includes: (C₁-C₄)alkyl, phenyl, phenyl(C₁-C₂)alkyl and cyclohexyl.
In this manner formula V compounds are prepared, wherein L is a hydrocarbyl sulphonate, a dihydrocarbyl phosphate or a N-arylcarbamate group, respectively.

The hydrindane intermediate of the above general formula VI is new. Therefore the present invention also relates to this intermediate, as well as to a method of preparing this compound.
A preferred hydrindane intermediate as defined above can be represented by the general formula 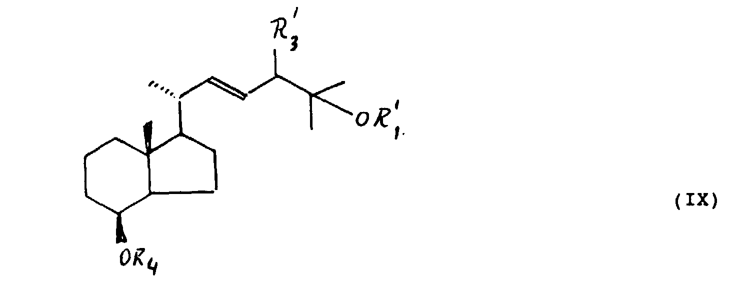 wherein:
R₁', R₃' and R₄ have the meanings given above.

Substantially pure C-24 enantiomeric vitamin D compounds can be obtained by using substantially pure hydrindane stereoisomers as intermediates. The present invention therefore also relates to a hydrindane intermediate of the above general formula IX, wherein the C-atom, to which substituent R₁' is attached, has either the **R** or the **S** configuration. As will be explained hereinafter, these isomers can be prepared stereospecifically in a convenient manner.

The hydrindane compound of the general formula VI, defined above, can be prepared conveniently by reacting a hydrindane compound of the general formula 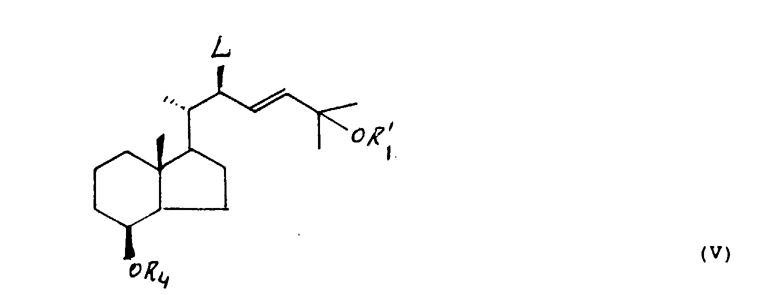
wherein L, R₁' and R₄ have the meanings given above,
with a reagent of the general formula

   R₃-MgX or R₃-Li

   wherein R₃ has the meaning given above and X is a halogen atom, in the presence of a Cu(I) compound.
Examples of suitable reagents for the above reaction are:
R₃-MgBr, R₃-MgI, R₃-MgCl and R₃-Li, wherein R₃ is defined above; examples of suitable cupro compounds are CuCN, CuCl and CuI.

It has been found, that the above-mentioned hydrindane stereoisomers of the general formula IX, wherein the C-atom, to which substiuent R₃' is attached, has either the **R** or the **S** configuration, can be prepared selectively by using as the starting substance a hydrindane compound of the general formula V having a specific leaving group L.
So a hydrindane intermediate of formula IX, wherein said C-atom has the configuration as shown in formula IXA below, can be prepared selectively from a hydrindane compound of the general formula 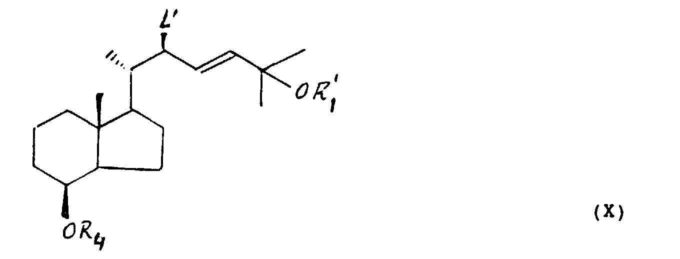 wherein:
R₁' and R₄ have the meanings above, and
L' is a di(hydrocarbyl)phosphate leaving group.

If, however, a hydrindane intermediate of formula IX is desired, wherein said C-atom has the configuration as shown in formula IXB below, as the starting substance a hydrindane compound can excellently be used, wherein L' is a N-arylcarbamate leaving group.

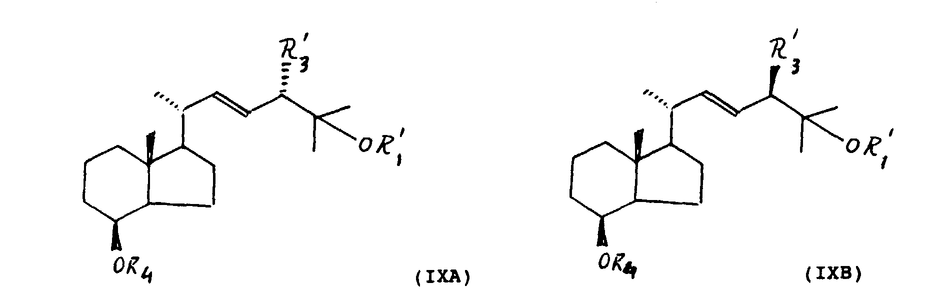

To improve the applicability of the new vitamin D compounds of the invention for the above-described pharmacotherapeutic indications, the compounds are usually processed to pharmaceutical compositions, comprising an effective amount of said vitamin D compound as the active ingredient in addition to a pharmaceutically acceptable carrier and/or at least one pharmaceutically acceptable auxiliary substance. Such a composition may be delivered in a dosage unit form for oral, topical (dermal) or parenteral administration, comprising approx. 0.1 µg to approx. 0.1 mg active ingredient per dosage unit.
A composition for diagnostic purposes may comprise, in addition to the vitamin D compound of the present invention, a compatible, non-toxic carrier and/or at least one auxiliary substance.
A cosmetical composition may comprise, in addition to an effective amount (in the range of approx. 0.1 µg to approx. 0.1 mg per dosage unit in a dosage unit form) of the vitamin D compound of the present invention, a cosmetically acceptable, non-toxic carrier and/or at least one auxiliary substance.

Finally the invention relates to a composition when for use in a method for the treatment and prophylaxis of a number of disease states including autoiummune diseases (including diabetes mellitus), acne, alopecia, skin aging (including photo-aging), imbalance in the immune system, inflammatory diseases such as rheumatoid arthritis and asthma, as well as diseases related to abnormal cell differentiation and/or proliferation, in a warm-blooded living being, comprising administering to said being or treating said being with a pharmaceutical composition as defined above in a quantity effective for the intended purpose. Examples of such diseases are psoriasis and other hyperproliferative skin diseases.
The present invention also relates to the use of the above pharmaceutical compositions for the treatment of solid, skin and blood cancers, in particular of blood cancers such as leukemia, of breast cancer, and of skin cancers such as melanoma and squamous cell carcinoma.
The above-defined cosmetical compositions, in particular selected from the group consisting of creams, lotions, ointments, liposomes and gels, can be used for the treatment and prevention of a number of skin disorders, such as inadequate skin firmness or texture, insufficient skin hydration, wrinkles and insufficient sebum secretion.

The invention will now be described in greater detail with reference to the following specific Examples.
The following abbreviations are used in the examples:
THF = tetrahydrofuran
TBS = tert.-butyl dimethyl silyl
MOM = methoxymethyl
TBAF = tert.-butyl ammonium fluoride
PPTS = pyridinium p-toluene sulphonate
PDC = pyridinium dichromate
AIBN = azodiisobutyronitrile
DCM = dichloromethane

### Examples

### Example I

### Preparation of hydrindane compounds, intermediates for modified vitamin D₂ compounds.

The reaction equations are presented in the Reaction Scheme A appended.
**(a). Preparation of compound 2.**
   Compound **1** is prepared as described by Sardina et al. in J. Org. Chem. 1986, **51**, 1264. A mixture of compound **1** (0.50 g, 3.90 mmol), n-Bu₃SnH (1.4 ml, 1.51 g, 5.20 mmol) and AIBN (20 mg) is irradiated with a tungsten lamp of 300 W for 15 min. and then heated at 95°C for 6 h. The reaction mixture is allowed to reach room temperature and is then chromatographed (silica gel; eluent: 0-7% EtOAc/hexane), producing compound **2** in a yield of 1.36 g, 83%, Rf=0.4 (10% EtOAc/hexane), liquid, b.p. 120°C/1 mm Hg.
   ¹H-NMR: 6.02 (2H, AB, J=19.5 Hz, HC=CH), 4.63 (2H, s, OCH₂O), 3.35 (3H, s, OCH₃), 1.47 (6H, m, Sn(CH₂₋)₃), 1.30 (6H, s, 2CH₃), 1.29 (6H, m, Sn(CH₂CH₂₋)₃), 0.91-0.85 (15H, m, (-CH₂CH₃)₃).
   ¹³C-NMR: 153.26 (C=), 126.58 (C=), 91.97 (OCH₂O), 77.76 (COMOM), 54.98 (OCH₃), 29.01 (SnCH₂₋)₃), 27.12 (Sn(CH₂CH₂₋)₃), 26.75 ((CH₃)₂), 13.55 ((-CH₃)₃), 9.42 ((-CH₂CH₃)₃).
**(b). Preparation of compound 6.**
   Compound **5** is prepared from compound **4** as described by Mascarenas et al. in J. Org. Chem. 1986, **51**, 1269. Compound **6** is prepared from compound **5** as described by Fernandez et al. in J. Org. Chem. 1992, **57**, 3173.
**(c). Preparation of compound 7.**
   A solution of n-BuLi in hexane (2.38 M, 3 ml, 7.11 mmol) is added dropwise to a solution of compound **2** (2.98 g, 7.11 mmol, freshly distilled) in 19 ml Et₂O, cooled at -80°C. The mixture is stirred for 4 hours and then cooled to -85°C. During the reaction compound 3 is intermediately formed in situ. Thereupon a solution of compound **6** (1.49 g, 4.59 mmol) in 36 ml Et₂O, cooled at -85°C, is added in 15 minutes. After stirring for 2 h, a drop of MeOH is added. The reaction is allowed to reach room temperature, after which a saturated NaCl solution (50 ml) and HCl (10%, 2 ml) are added. Extraction with Et₂O (2x30 ml), drying, filtration and concentration yields a residue which is chromatographed over a silica gel column; eluent: 10% EtOAc/hexane. Products **7** [1.58 mg, 76%, Rf=0.61 (30% EtOAc/hexane); crystallizing upon cooling, m.p. 49°C] and **7a** [0.24 mg, 11%, Rf=0.57 (30% EtOAc/hexane)] are obtained. Compound **7** is identified by ¹H-NMR and ¹³C-NMR.
   ¹H-NMR: 5.66 (1H, d, J=16.1 Hz, H-24), 5.58 (1H, dd, J=16.1 and 3.5 Hz, H-23), 4.62 (2H, s, OCH₂O), 4.25 (1H, s, H-22), 3.98 (1H, s, H-8), 3.33 (3H, s, OCH₃), 1.31 (6H, s, CH₃-26 and 27), 0.90 (3H, s, CH₃-18), 0,86 (9H, s, SiC(CH₃)₃), 0.82 (3H, d, J=5.93 Hz, CH₃-21), -0.01 (3H, s, SiCH₃), -0.03 (3H, s, SiCH₃).
   ¹³C-NMR: 135.00 (C=), 132.58 (C=), 91.75 (OCH₂O), 75.88 (C-25), 73.35, 69.41, 54.95 (OCH₃), 53.13, 52.98, 41.95 (C-13), 40.81, 40.60 (CH₂), 34.35 (CH₂), 27.32 and 27.24 (C-26 and C-27), 26.81 (CH₂), 25.74 (SiC(CH₃)₃), 22.93 (CH₂), 17.94 (SiC(CH₃)₃), 17.58 (CH₂), 13.57, 11.78, -4.89 (SiCH₃).
**(d). Preparation of compound 8.**
   A solution of n-BuLi in hexane (2.38 M, 1.26 ml, 3.0 mmol) is added to a solution of compound **7** (1.24 g, 2.71 mmol) in 25 ml Et₂O while stirring and cooling at -78°C. After 15 min, ClPO(OEt)₂ (0.43 ml, 0.52 g, 3.0 mmol, distilled over P₂O₅) is added. The mixture is stirred for 3 h, and then poured onto a mixture of saturated aqueous NaCl (100 ml) and ice (50 ml). The mixture is extracted with Et₂O (3x40 ml); the organic layer is dried, filtered, concentrated and chromatographed over a silica gel column (eluent: 15-30% EtOAc/hexane), to produce compound **8** in a yield of 1.50 g [93%, Rf=0.3 (30% EtOAc/hexane)].
   ¹H-NMR: 5.71 (1H, d, J=15.95 Hz, H-24), 5.60 (1H, dd, J=15.95 and 5.0 Hz, H-23), 4.89 (1H, t, J=6.0 Hz, H-22), 4.64 (2H, s, OCH₂O), 4.14-3.97 (4H, m, J=7.2 Hz, P(OCH₂CH₃)₂), 3.97 (1H, s, H-8), 3.34 (3H, s, OCH₃), 1.35-1.26 (6H, m, P(OCH₂CH₃)₂), 1.32 (6H, s, CH₃-26 and 27), 0.91 (3H, d, J=6.33 Hz, CH₃-21), 0,90 (3H, s, CH₃-18), 0.87 (9H, s SiC(CH₃)₃), -0.01 (3H, s, SiCH₃), -0.02 (3H, s, SiCH₃).
   ¹³C-NMR: 137.50, 128.04, 91.74 (OCH₂O), 81.16 (d, J=6.9 Hz, C-22), 75.50 (C-25), 69.23, 63.23 (c, J=2.8, P(OCH₂CH₃)₂), 54.85 (OCH₃), 52.92, 52.53, 41.91 (C-13), 41.83, 40.49 (CH₂), 34.18 (CH₂), 27.06 (C-26 and 27), 26.75 (CH₂), 25,59 (SiC(CH₃)₃), 22.84 (CH₂), 17.79 (SiC(CH₃)₃), 17.41 (CH₂, 15.95 (c, J=3.5 Hz, P(OCH₂CH₃)₂), 13.32, 12.16, -5.04 (SiCH₃), -5.40 (SiCH₃).

### Example II

### Preparation of compound 25 from compound 8.

The reaction equations are presented in the Reaction Scheme B appended.
**(a). Preparation of compound 9.**
   A mixture of CuCN (0.90 g, 10 mmol) and LiCl (0.85 g, 20 mmol) in 28 ml THF is stirred at room temp. until a homogeneous solution is obtained. After cooling to -78°C, a solution of n-BuMgCl in Et₂O (2M, 5 ml, 10 mmol) is added. The resulting reaction mixture is stirred for 5 min. A solution of phosphate **8** (0.96 g, 1.62 mmol, dried over P₂O₅) in 32 ml THF is added. The reaction mixture is stirred for 10 h, after which the mixture is allowed to reach room temperature without removing the cooling bath (12 h). After dropwise addition of a saturated solution of NH₄Cl (30 ml), the mixture is stirred for 10 min and then extracted with hexane (3x50 ml). The organic phase is dried, filtered and concentrated, yielding 784 mg of compound **9** [98%, Rf=0.8 (10% EtOAc/hexane].
   ¹H-NMR: 5.20 (1H, dd, J=15.2 and 8.5 Hz, H-22 or H-23), 5.03 (1H, dd, J=15.2 and 9.2 Hz, H-22 or H-23), 4.69 (2H, s, OCH₂O), 3.98 (1H, s, H-8), 3.36 (3H, s, OCH₃), 1.16 (3H, s, CH₃-26 or 27), 1.11 (3H, s, CH₃-26 or 27), 0.98 (3H, d, J=6.59 Hz, CH₃-21), 0.92 (3H, s, CH₃-18), 0.88 (12H, s and t, SiC(CH₃)₃ and (CH₂)₃CH₃), 0.00 (3H, s, SiCH₃), -0.01 (3H, s, SiCH₃).
   ¹³C-NMR: 139.75 (C=), 128.14 (C=), 90.91 (OCH₂O), 78.31 (C-25), 69.51, 56.56, 55.08, 53.17, 42.03 (C-13), 40.67 (CH₂), 39.98, 34.45 (CH₂), 30.17 (CH₂), 28.26 (CH₂), 27.86 (CH₂), 25.76 (SiC(CH₃)₃), 25.52, 23.12 (CH₂), 23.06, 22.65 (CH₂), 20.69, 17.98 (SiC(CH₃)₃), 17.64 (CH₂), 14.07, 13.88, -4.87 (SiCH₃), -5.24 (SiCH₃).
**(b). Preparation of compound 13.**
   Compound **9** is added to a solution of TBAF in THF (1.1 M, 6 ml, 6.6 mmol). The reaction mixture is stirred for 24 h at 70°C and 48 h at room temperature. The reaction mixture is then poured into a saturated aqueous solution of NaHCO₃ (100 ml) and extracted with Et₂O (3x30 ml). The combined ether layers are dried, filtered and concentrated. The residue is chromatographed over silica gel (eluent: 9% EtOAc/hexane), yielding 471 mg of compound **13** [80%, Rf=0.4 (15% EtOAc/hexane)].
   ¹H-NMR: 5.18 (1H, dd, J=15.18 and 8.39 Hz, H-22 or H-23), 5.03 (1H, dd, J=15.18 and 9.10 Hz, H-23 or H-22), 4.67 (2H, s, OCH₂O), 4.04 (1H, s, H-8), 3.33 (3H, s, OCH₃), 1.14 (3H, s, CH₃-26 or 27), 1.09 (3H, s, CH₃-26 or 27), 0.97 (3H, d, J=6.61 Hz, CH₃-21), 0.92 (3H, s, CH₃-18), 0.84 (3H, t, J=6.92 Hz, (CH₂)₃CH₃).
   ¹³C-NMR: 139.46 (C=), 128.32 (C=), 90.87 (OCH₂O), 78.26 (C-25), 69.35, 56.38, 55.07, 53.12, 52.69, 41.71 (C-13), 40.30 (CH₂), 39.92, 33.57 (CH₂), 30.15 (CH₂), 28.19 (CH₂), 27.70 (CH₂), 25.49, 23.06, 22.61 (CH₂), 22.53 (CH₂), 20.61, 17.38 (CH₂), 14.05, 13.64.
**(c). Preparation of compound 17.**
   To a solution of 196 mg (0.517 mmol) of compound **13** in 6 ml DCM are added 0.58 g (1.55 mmol) PDC and approx. 5-10 mg PPTS. After stirring at room temp. for 3.5 h, 20 ml Et₂O is added. Separation of the organic phase, filtration over silica gel and evaporation yields 0.18 g of a residue, which after flash chromatography (silica gel; eluent: EtOAc/hexane 1:20) gives the desired product **17** in a yield of 175.5 mg (90%).
**(d). Preparation of protected vitamin D compound 21.**
   To a solution of the phosphine-oxide **45** (370 mg, 0.63 mmol) in 5 ml dry THF at -78°C is added a small amount of n-BuLi in THF (2.5 M, approx. 230 µl) until the reaction mixture colours red. Thereupon n-BuLi in THF (2.5 M, 254 µl, 0.63 mmol) is added dropwise. After stirring for 5 min, a solution of 80.1 mg (0.21 mmol) of compound **17** in 2 ml dry THF is added. The mixture is stirred for 0.5 h and allowed to reach room temperature. Purification by flash- chromatography yields 53.4 mg (34%) of the desired title compound **21**.
**(e). Preparation of vitamin D compound 25.**
   Compound **21** is dissolved in a 0.83 M solution of TBAF (2.3 ml, 1.88 mmol, 20 eq.) in THF. The mixture is stirred overnight at ambient temperature. The solvent is evaporated and the residue is taken up with Et₂O and washed with brine. The organic phase is dried over MgSO₄ and the solvent evaporated. The residue is quickly chromatographed over silica (Et₂O) and the resulting product is dissolved in methanol (7 ml) and 1.15 g of Dowex^{R} resin (acidic resin) AG 50W-X4 (washed with MeOH (4x10 ml)) is added. After stirring overnight, filtration and washing with EtOAc (3x20 ml), the reaction mixture is evaporated. The desired vitamin D compound **25** is obtained in a yield of 20 mg as a white solid.

### Example III

### Preparation of compound 26 from compound 8.

The reaction equations are presented in the Reaction Scheme B appended. Via a reaction sequence analogous to that described in Example II, Vitamin D compound **26** is prepared from compound **8**, via the intermediate compounds **10**,**14**, **18** and **22.**

Compound **10** is identified by ¹H-NMR and ¹³C-NMR:
¹H-NMR: 5.25 (1H, dd, J=15.2 and 8.1 Hz, H-22 or H-23), 5.14 (1H, dd, J=15.2 and 7.9 Hz, H-23 or H-22), 4.73 (2H, s, OCH₂O), 3.99 (1H, s, H-8), 3.37 (3H, s, OCH₃), 1.25 (3H, s, CH₃-26 or 27), 1.24 (3H, s, CH₃-26 or 27), 0.98 (3H, d, J=6.58 Hz, CH₃-21), 0.92 (3H, s, CH₃-18), 0.88 (9H, s, SiC(CH₃)₃), 0.92-(-0.04) (5H, 5m, c-Pr), 0.00 (3H, s, SiCH₃), -0.01 (3H, s, SiCH₃).
¹³C-NMR: 138.95 (C=), 126.64 (C=), 90.93 (OCH₂O), 78.86 (C-25), 69.94, 56.75, 56.48, 54.93, 53.19, 41.99 (C-13), 40.68 (CH₂), 40.00, 34.44 (CH₂), 27.85 (CH₂), 25.76 (SiC(CH₃)₃), 24.88 (C-26 or C-27), 24.73 (C-26 or C-27), 23.14 (CH₂), 20.69, 17.94 (SiCCH₃)₃), 17.64 (CH₂), 13.90, 11.14, 5.49 (CH₂(c-Pr)), 2.41 (CH₂(c-Pr)), -4.89 (SiCH₃), -5.26 (SiCH₃).

Compound **14** is identified by ¹H-NMR and ¹³C-NMR:
¹H-NMR: 5.27 (1H, dd, J=15.22 and 8.42 Hz, H-22 or H-23), 5.14 (1H, dd, J=15.22 and 8.18 Hz, H-23 or H-22), 4.72 (2H, s, OCH₂O), 4.06 (1H, s, H-8), 3.36 (3H, s, OCH₃), 1.24 (6H, s, CH₃-26 and 27), 0.98 (3H, d, J=6.60 Hz, CH₃-21), 0.93 (3H, s, CH₃-18), 0.92-(-0.06) (5H, 5m, c-Pr).
¹³C-NMR: 138.61 (C=), 126.79 (C=), 90.84 (OCH₂O), 74.84 (C-25), 69.20, 56.68, 56.26, 54.92, 52.67, 41.65 (C-13), 40.26 (CH₂), 39.87, 33.52 (CH₂), 27.58 (CH₂), 24.79 and 24.71 (C-26 and C-27), 22.50 (CH₂), 20.59, 17.34 (CH₂), 13.59, 11.09, 5.52 and 2.39 (CH₂CH₂(c-Pr)).

Compound **26** is identified by ¹H-NMR and ¹³C-NMR:
¹H-NMR (δ, CD₂Cl₂): 6.23 (1H, d, J=11.2 Hz), 6.00 (1H, d, J=11.2 Hz), 5.30-5.27 (2H, m), 5.26 (1H, s), 4.94 (1H, s), 4.36 (1H, dd, J=4.4 and 6.5 Hz), 4.15 (1H, m), 2.82 (1H, d, J=12.5 Hz), 2.53 (1H, dd, J=2.66 and 13.3 Hz), 2.25 (1H, dd, J=6.5 and 13.3 Hz), 1.19 (3H, s), 1.16 (3H, s), 1.02 (3H, d, J=6.6 Hz), 0.73 (1H, m), 0.55 (3H, s), 0.53 (1H, m), 0.36 (1H, m), 0.22 (1H, m), 0.02 (1H, m).
¹³C-NMR (δ, CD₂Cl₂): 148.41, 143.12, 140.28, 133.90, 127.22, 124.83, 117.49, 111.74, 73.29, 71.04, 67.06, 58.89, 56.72, 56.48, 46.11, 45.66, 43.28, 40.94, 40.72, 29.34, 28.24, 27.84, 27.41, 23.93, 22.63, 21.23, 12.33, 11.67, 6.11, 2.54.

### Example IV

### Preparation of compound 27a from compound 8.

The reaction equations are presented in the Reaction Scheme B appended. Vitamin D compound **27a** is prepared from compound **8**, via the intermediate compounds **11**, **15**, **19**, **23** and **27**, analogous to the reaction sequence described in Example II for the first 5 steps. During the fifth step (deprotection step) ring closure takes place between the deprotected aldehyde group of the 24-substituent and the free 25-OH group, forming compound **27**. Compound **27** can easily be converted to the corresponding alcohol **27a** by ring-opening and reduction of the aldehyde group.

Compound **11** is identified by ¹H-NMR and ¹³C-NMR:
¹H-NMR: 5.22 (1H, dd, J=15.2 and 8.5 Hz, H-22 or H-23), 5.04 (1H, dd, J=15.2 and 9.2 Hz, H-23 or H-22), 4.83 (1H, t, J=4.5 Hz, OCHRO), 4.72 (2H, AB, J=7.4 Hz, OCH₂O), 3.96 (1H, s, H-8), 3.96-3.78 (4H, m, OCH₂CH₂O), 3.34 (3H, s, OCH₃), 1.16 (3H, s, CH₃-26 or 27), 1.11 (3H, s, CH₃-26 or 27), 0.96 (3H, d, J=6.57 Hz, CH₃-21), 0.90 (3H, s, CH₃-18), 0.87 (9H, s, SiC(CH₃)₃), -0.01 (3H, s, SiCH₃), -0.03 (3H, s, SiCH₃).
¹³C-NMR: 140.33 (C=), 127.58 (C=), 104.91 (OCHRO), 90.89 (OCH₂O), 78.10 (C-25), 69.45, 64.78 (OCH₂CH₂O), 64.73 (OCH₂CH₂O), 56.41, 55.09, 53.25, 53.10, 41.99 (C-13), 40.62 (CH₂), 39.94, 34.39 (CH₂), 32.46 (CH₂), 27.93 (CH₂), 25.73 (SiC(CH₃)₃), 25.47, 23.14 (CH₂), 23.03 (CH₂), 22.94, 20.56, 17.93 (SiC(CH₃)₃), 17.60, (CH₂), 13.83, -4.91 (SiCH₃), -5.28 (SiCH₃).

Compound **27** is identified by ¹H-NMR and ¹³C-NMR:
¹H-NMR (δ, CD₂Cl₂): 6.33 (1H, d, J=11.2 Hz), 5.99 (1H, d, J=11.2 Hz), 5.30-5.26 (1H, m), 5.27 (1H, s), 5.12 (1H, dd, J=15.3 Hz and 8.4 hz), 4.93 (1H, s), 4.60 (0.375 H, d, J=1.4 hz), 4.44 (0.625 H, dd, J=9.5 and 2.4 Hz), 4.35 (1H, m), 4.14 (1H, m), 3.34 (1.875 H, s), 3.32 (1.125 H, s), 2.82 (1H, d, J=12.4 Hz), 2.52 (1H, dd, J=13.4 and 3.5 Hz), 2.25 (1H, dd, J=13.4 and 6.6 hz), 1.17 (3H, s), 1.12 (1.125 H, s), 1.08 (1.875 H, s), 0.99 (3H, d, J=6.6 Hz), 0.53 (3H, s).
¹³C-NMR (δ, CD₂Cl₂): 148.45, 143.01, 142.98, 138.77, 138.19, 134.02, 133.99, 129.05, 128.46, 124.76, 117.53, 111.70, 99.08, 98.41, 76.26, 75.47, 70.95, 67.03, 56.70, 56.65, 55.59, 55.14, 49.12, 48.68, 46.13, 45.63, 43.32, 40.77, 40.72, 40.69, 31.53, 30.40, 29.74, 29.70, 29.35, 28.05, 25.91, 23.92, 23.35, 22.58, 22.11, 20.99, 19.66, 12.34.

### Example V

### Preparation of compound 28 from compound 8.

The reaction equations are presented in the Reaction Scheme B appended. Via a reaction sequence analogous to that described in Example II, Vitamin D compound **28** is prepared from compound **8**, via the intermediate compounds **12**, **16**, **20** and **24**.

Compound **12** is identified by ¹H-NMR and ¹³C-NMR:
¹H-NMR: 5.23 (1H, dd, J=15.2 and 9.36 Hz, H-22 or H-23), 5.13 (1H, dd, J=15.2 and 8.3 Hz, H-22 or H-23), 4.68 (2H, s, OCH₂O), 3.99 (1H, s, H-8), 3.35 (3H, s, OCH₃), 1.17 (6H, s, CH₃-26 and 27), 0.99 (3H, d, J=6.55 Hz, CH₃-21), 0.93 (3H, 9, CH₃-18), 0.88 (9H, 9, SiC(CH₃)₃), 0.87 and 0.84 (6H, 2d, J=3.5 and 3.4 Hz, CH(CH₃)₂), 0.00 (3H, s, SiCH₃), -0.01 (3H, s, SiCH₃).
¹³C-NMR: 140.65 (C=), 124.53 (C=), 90.84 (OCH₂O), 78.69 (C-25), 69.49, 58.64, 56.48, 55.05, 53,20, 42.00 (C-13), 40.69 (CH₂), 40.35, 34.45 (CH₂), 28.38 (CH₂), 26.75, 26.15, 25,77 (SiC(CH₃)₃), 24.48, 24.09, 23.19 (CH₂), 20.71, 18.96, 17.96 (SiC(CH₃)₃), 17.64 (CH₂), 13.88, -4.89 (SiCH₃), -5.25 (SiCH₃).

Compound **28** is identified by ¹H-NMR and ¹³C-NMR:
¹H-NMR (δ, CD₂Cl₂): 6.34 (1H, d, J=11.2 Hz), 6.00 (1H, d, J=11.2 Hz), 5.30-5.27 (2H, m), 5.26 (1H, s), 4.94 (1H, dd, J=1.2 and 1.8 hz), 4.36 (1H, m), 4.14 (1H, m), 2.82 (1H, d, J=12.3 Hz), 2.54 (1H, dd, J=3.5 and 13.4 Hz), 2.25 (1H, dd, J=6.6 and 13.4 Hz), 1.14 (3H, s), 1.04 (3H, d, J=6.6 Hz), 0.86 (6H, d, J=6.9 Hz), 0.56 (3H, s).
¹³C-NMR (δ, CD₂Cl₂): 148.43, 1543.13, 142.30, 133.86, 124.85, 124.68, 117.48, 111.69, 72.95, 71.06, 67.08, 60.30, 56.74, 56.54, 46.13, 45.69, 43.32, 41.27, 40.74, 29.34, 28.75, 28.57, 28.38, 27.70, 24.39, 23.93, 22.67, 21.26, 19.00, 12.27.

### Example VI

### Preparation of compound 30 from compound 17.

The reaction equations are presented in the Reaction Scheme B appended.
**(a). The preparation of protected 19-nor-vitamin D compound 29.**
   To a solution of the phosphine-oxide **46** (360 mg, 0.63 mmol) in 5 ml dry THF at -78°C is added a small amount of n-BuLi in THF (2.5 M, approx. 230 µl) until the reaction mixture colours red. Thereupon n-BuLi in THF (2.5 M, 254 µl, 0.63 mmol) is added dropwise. After stirring for 5 min, a solution of 80.1 mg (0.21 mmol) of compound **17** in 2 ml dry THF is added. The mixture is stirred for 0.5 h and allowed to reach room temp. Purification by chromatography yields 106.7 mg (69%) of the desired title compound **29**.
**(b). The preparation of 19-nor-vitamin D compound 30.**
   Compound **29** (103 mg, 0.139 mmol) is dissolved in a 0.83 M solution of TBAF (3.4 ml, 2.78 mmol, 20 eq.) in THF. The mixture is stirred overnight at ambient temperature. The solvent is evaporated and the residue is taken up with Et₂O and washed with brine. The organic phase is dried over MgSO₄ and the solvent evaporated. The residue is quickly chromatographed over silica (Et₂O) and the resulting product is dissolved in methanol (10 ml) and 1.75 g of Dowex^{R} resin (acidic resin) AG 50W-X4 (washed with MeOH (4x12 ml)) is added. After stirring overnight, filtration and washing with EtOAc (3x20 ml), the reaction mixture is evaporated. The desired 19-nor-vitamin D compound **30** is obtained in a yield of 30 mg as a white solid. The product is identified by ¹H-NMR and ¹³C-NMR .
   ¹H-NMR (δ, CDCl₃): 0.36 (3H, s, 18-CH₃), 0.87 (3H, t, CH₃), 1.05 (3H, d, 21-CH₃), 1.12 (3H, s, 26-CH₃), 1.17 (3H, s, 27-CH₃), 2.48 (1H, dd), 2.71 (1H, dd,), 2.80 (1H, bd), 4.00-4.16 (2H, m), 5.10 (1H, dd, 22-H), 5.37 (dd, 23-H), 5.85 (1H, d, H-6), 6.31 (1H, d, H-7).
   ¹³C-NMR: 12.32, 14.10, 21.20, 22.35, 22.55, 23.47, 26.80, 26.99, 28.07, 28.91, 29.19, 30.40, 37.20, 40.36, 40.67, 42.21, 44.71, 45.70, 54.85, 56.19, 56.35, 67.22, 67.43, 72.14, 115.37, 123.80, 128.00, 131,26, 141.49, 142.87.

### Example VII

### Preparation of compound 30 and 31 from compound 8.

The reaction equations are presented in the Reaction Scheme C appended.

A solution of phenylmagnesiumchloride in THF (2M, 15 ml, 30 mmol) is added to a solution of CuCN (2.69 g, 30 mmol) and LiCl (2.54 g, 40 mmol) in 40 ml THF while stirring and cooling at -78°C. The reaction mixture is stirred for 5 min and a solution of phosphate **8** (897 mg, 1.518 mmol, dried over P₂O₅) in 15 ml THF is added. The reaction is stirred for 2 days under exclusion of light. To the reaction mixture is added a saturated solution of NH₄cl (100 ml). After stirring for 10 min, the mixture is extracted at room temp with Et₂O (3x60 ml). The combined organic layers are dried, filtered and concentrated. The residue is separated by chromatograpy over silica gel (eluent: 0-1.5% EtOAc/hexane), yielding 173 mg of compound **30** [22%, Rf=0.52 (5% EtOAc/hexane), liquid], and 591 mg of compound **31** [76%, Rf=0.46 (5%EtOAc/hexane), m.p. 71-72°C]. The compounds **30** and **31** are identified by ¹H-NMR and ¹³C-NMR.

Compound **30**: ¹H-NMR (δ): 7.26-7.17 (5H, m, Ar), 5.83 (1H, dd, J=15.3 and 8.8 Hz, H-23), 5.28 (1H, dd, J=15.3 and 8.4 Hz, H-22), 4.69 (2H, AB, J=7.4 Hz, OCH₂O), 3.96 (1H, s, H-8), 3.30 (3H, s, OCH₃), 3.20 (1H, d, J=8.8 Hz, H-24), 1.21 (3H, 9, CH₃-26 or 27), 1.15 (3H, s, CH₃-26 or 27), 1.00 (3H, d, J=6.59 Hz, CH₃-21), 0.91 (3H, s, CH₃-18), 0.88 (9H, s, SiC(CH₃)₃), 0.00 (3H, s, SiCH₃), -0.02 (3H, s, SiCH₃).
¹³C-NMR (δ): 142.65 (C), 139.25 (C=), 129.70 (C=), 127.60 (C=), 127.19 (C=), 125.95 (C=), 91.01 (OCH₂O), 78.08 (C-25), 69.44, 59.89, 56.66, 54.95, 53.04, 42.03 (C-13), 40.62 (CH₂), 39.77, 34.40 (CH₂), 27.58 (CH₂), 25.75 (SiC(CH₃)₃), 25.02, 24,98, 23.01 (CH₂), 20.26, 17.93 (SiC(CH₃)₃), 17.61 (CH₂), 13.83, -4.90 (SiCH₃), -5.27 (SiCH₃).

Compound **31**: ¹H-NMR (δ): 7.31-7.15 (5H, m, Ar), 5.92 (1H, dd, J=15.7 and 10.1 Hz, H-23), 5.60 (1H, d, J=15.7 Hz, H-24), 4.67 (2H, s, OCH₂O), 4.00 (1H, s, H-8), 3.51 (1H, d, J=10.1 Hz, H-22), 3.36 (3H, s, OCH₃), 1.39 (3H, s, CH₃-26 or 27), 1.37 (3H, 9, CH₃-26 or 27), 0,94 (3H, s, CH₃-18), 0.89 (9H, s, SiC(CH₃)₃), 0,76 (3H, d, J=6.76 Hz, CH₃-21), 0.01 (6H, s, Si(CH₃)₂).
¹³C-NMR (δ): 145.22 (C), 138.09 (C=), 128.08 (C=), 127.92 (C=), 127.43 (C=), 125.64 (C=), 91.92 (OCH₂O), 76.24 (C-25), 69.44, 54.97, 53.10, 50.01, 48.54, 42.03, 40.71 (CH₂), 34.31 (CH₂), 27.91, 27.26 (CH₂), 27.22, 25.76 (SiC(CH₃)₃), 23.04 (CH₂), 17.96 (SiC(CH₃)₃), 17.55 (CH₂), 13.72, 13.29, -4.87 (SiCH₃), -5.25 (SiCH₃).

### Example VIII

### Preparation of compound 36 from compound 30.

The reaction equations are presented in the Reaction Scheme C appended.

Via a reaction sequence analogous to that described in Example II, Vitamin D compound **36** is prepared from compound **30**, via the intermediate compounds **33**, **34** and **35**.

Compound **36** is identified by ¹H-NMR and ¹³C-NMR:
¹H-NMR (δ, CD₂Cl₂): 7.29-7.18 (5H, m), 6.33 (1H, d, J=11.2 Hz), 5.98 (1H, d, J=11.2 Hz), 5.82 (1H, dd, J=15.2 and 9.4 hz), 5.42 (1H, dd, J=15.2 and 8.5 Hz), 5.26 (1H, s), 4.92 (1H, dd, J=2.0 and 1.2 Hz), 4.34 (1H, m), 4.14 (1H, m), 3.18 (1H, d, J=9.5 Hz), 1.13 (3H, s), 1.10 (3H, s), 1.06 (3H, d, J=6.6 Hz), 0.53 (3H, s).
¹³C-NMR (δ, CD₂Cl₂): 148.41, 142.99, 142.44, 140.46, 133.96, 129.58, 128.32, 127.19, 126.62, 124.78, 117.52, 111.784, 72.69, 71.00, 67.03, 60.72, 56.62, 46.17, 45.65, 43.29, 40.70, 40.66, 31.93, 29.33, 28.13, 27.99, 27.80, 23.91, 23.00, 22.56, 20.86, 14.23, 12.29.

### Example IX

### Preparation of compound 43 from compound 31.

The reaction equations are presented in the Reaction Scheme C appended.

Via a reaction sequence analogous to that described in Example II, Vitamin D compound **43** is prepared from compound **31**, via the intermediate compounds **37**, **39** and **41**.

Compound **43** is identified by ¹H-NMR and ¹³C-NMR:
¹H-NMR (δ, CD₂Cl₂): 7.27-7.14 (5H, m), 6.35 (1H, d, J=11.2 Hz), 6.03 (1H, d, J=11.2 Hz), 5.89 (1H, dd, J=15.8 and 10.3 Hz), 5.55 (1H, d, J=15.8 Hz), 5.29 (1H, d, J=1.4 Hz), 4.96 (1H, s), 4.36 (1H, m), 4.15 (1H, m), 3.5 (1H, d, J=10.3 Hz), 2.80 (1H, d, J=12 Hz), 2.52 (1H, d, J=13 Hz), 2.25 (1H, dd, J=13 and 6.5 hz), 1.29 (3H, s), 1.27 (3H, s), 0.80 (3H, d, J=6.8 Hz), 0.57 (3H, s).
¹³C-NMR (δ, CD₂Cl₂): 148.43, 145.66, 142.99, 138.69, 133.96, 128.33, 128.27, 127.51, 125.92, 124.84, 117.61, 111.76, 75.20, 71.08, 67.08, 56.74, 55.13, 50.67, 50.50, 46.14, 45.68, 43.34, 43.20, 40.90, 29.32, 27.97, 26.78, 25.77, 23.89, 22.64, 13.69, 12.11.

### Example X

### Preparation of compound 44 from compound 8.

The reaction equations are presented in the Reaction Scheme C appended.

**(a). Preparation of compound 32.**

A solution of vinylmagnesiumchloride in THF (15%, 5.08 ml, 8.6 mmol) is added to a solution of CuCN (770 mg, 8.6 mmol) and LiCl (729 mg, 17.2 mmol) in 20 ml THF while stirring and cooling at -78°C. The reaction mixture is stirred for 5 min and a solution of phosphate **8** (497 mg, 0.841 mmol, dried over P₂O₅) in 20 ml THF is added. The reaction is stirred for 3 days under exclusion of light. To the reaction mixture is added a satd. solution of NH₄Cl (25 ml). After stirring for 5 min, the mixture is extracted at room temp with Et₂O (3x20 ml). The combined organic layers are dried, filtered and concentrated. The residue is chromatographed over silica gel (eluent: 2-30% EtOAc/hexane), yielding 225 mg of compound **32** [58%, Rf=0.8 (10% EtOAc/hexane), crystallizing upon cooling, m.p. 30°C], whereas 61 mg of starting compound **8** is recovered. Compound **32** is identified by ¹H-NMR and ¹³C-NMR.
¹H-NMR (δ): 5.83 (1H, ddd, J=17.3, 10.4 and 5.3 Hz, CH=CH₂), 5.57-5.44 (2H, m, H-23 and H-24), 5.01 (1H, dt, J_{cis}=10.4 Hz, J_{gem}=1.8 Hz, C=CHH), 4.91 (1H, dt, Jₜᵣₐₙₛ=17.3 Hz, J_{gem}=1.8 Hz, C=CHH), 4.65 (2H, s, OCH₂O), 3.98 (1H, s, H-8), 3.35 (3H, s, OCH₃), 2.86 (1H, s, H-22), 1.34 (6H, s, CH₃-26 and 27), 0.92 (3H, s, CH₃-18), 0.88 (H, s, SiC(CH₃)₃), 0.84 (3H, d, J=6.81 Hz, CH₃-21), 0.00 (3H, s, SiCH₃), -0.01 (3H, s, SiCH₃).
¹³C-NMR (δ): 142.31 (C=), 137.57 (C=), 127.93 (C=), 114.31 (=CH₂), 91.86 (OCH₂O), 76.20 (C-25), 69.43, 54.96, 54.73, 53.01, 48.54, 42.01 (C-13), 40.72 (CH₂), 40.35, 34.33 (CH₂), 27.82, 27.24, 26.90 (CH₂), 25.74 (SiC(CH₃)₃), 23.00 (CH₂), 17.94 (SiC(CH₃)₃), 17.56 (CH₂), 13.86, 13.69, -4.89 (SiCH₃), -5.27 (SiCH₃).

Via a reaction sequence analogous to that described in Example II, Vitamin D compound **44** is prepared from compound **32**, via the intermediate compounds **38**, **40** and **42**.

Compound **44** is identified by ¹H-NMR and ¹³C-NMR:
¹H-NMR (δ, CD₂Cl₂): 6.33 (1H, d, J=11.2 Hz), 6.00 (1H, d, J=11.2 Hz), 5.85 (1H, ddd, J=17.2, 10.4 and 5.7 Hz), 5.48 (1H, dd, J=15.9 and 8.4 Hz), 5.42 (1H, d, J=15.9 Hz), 5.28 (1H, t, J=1.6 Hz), 5.00 (1H, dt, J=10.4 and 1.8 Hz), 4.94 (1H, s), 4.93, (1H, dt, J=17.2 and 1.8 Hz), 4.35 (1H, m), 4.14 (1H, m), 2.85 (1H, m), 2.82 (1H, d, J=12 Hz), 2.53 (1H, dd, J=13 and 2.6 Hz), 2.25 (1H, dd, J=13.2 and 6.6 Hz), 1.24 (3H, s), 1.33 (3H, s), 0.87 (3H, d, J=6.8 Hz), 0.55 (3H, s).
¹³C-NMR (δ, CD₂Cl₂): 148.41, 143.06, 142.83, 138.19, 133.90, 127.95, 124.85, 117.54, 114.32, 111.77, 75.16, 71.09, 67.07, 56.63, 54.88, 50.39, 49.19, 46.08, 45.69, 43.33, 41.54, 40.89, 29.33, 27.63, 26.64, 25.84, 23.91, 22.60, 14.26, 12.08.

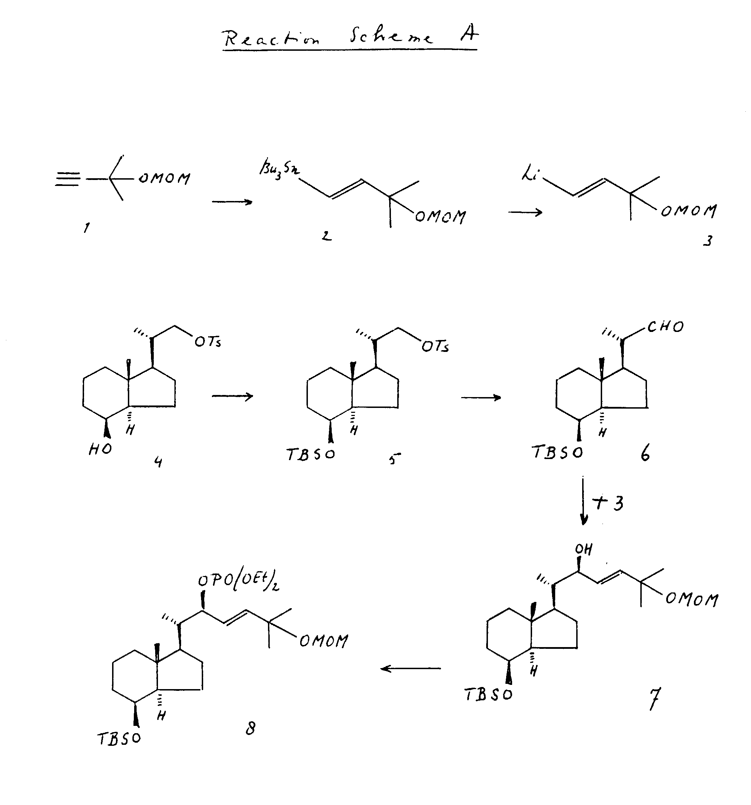

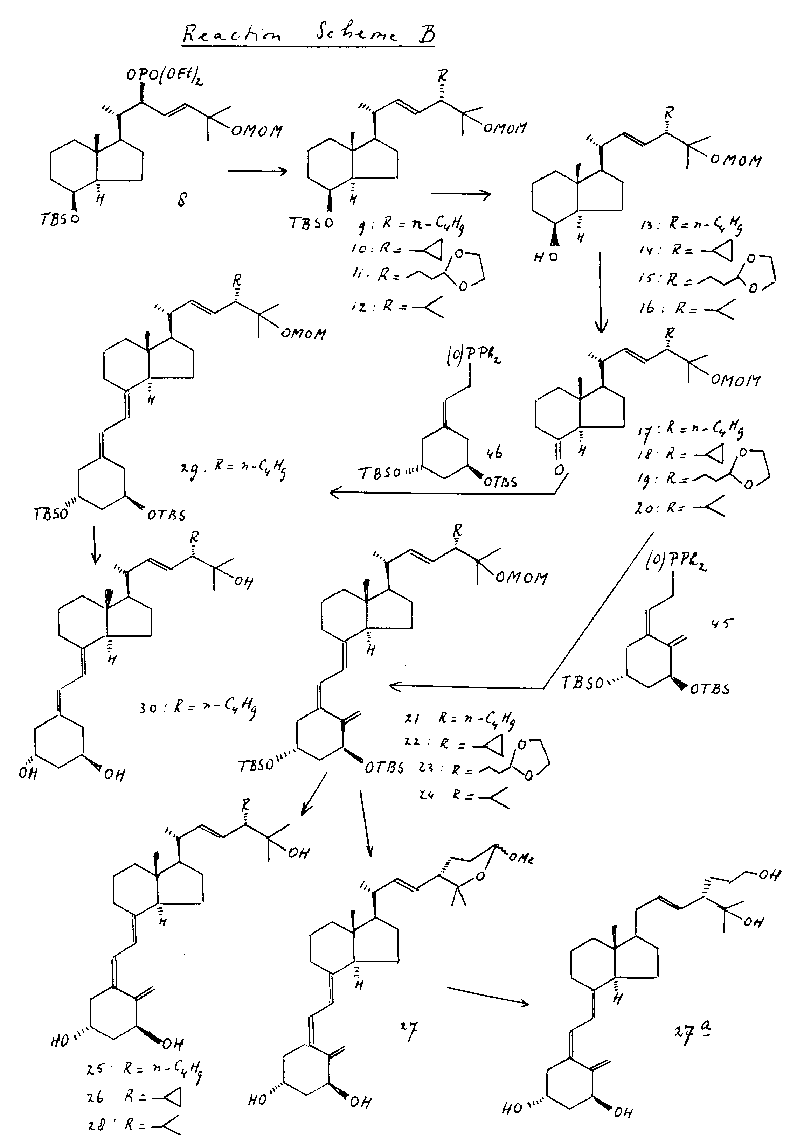

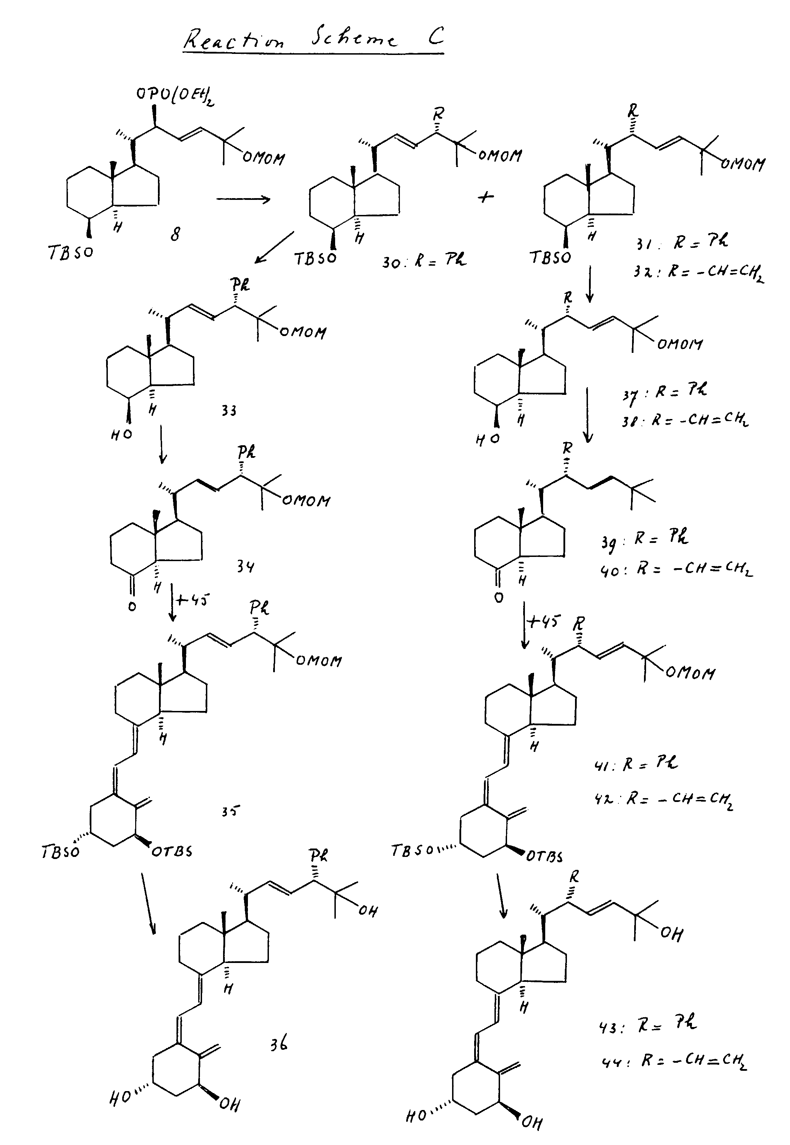

## Claims

1. A vitamin D compound of the general formula 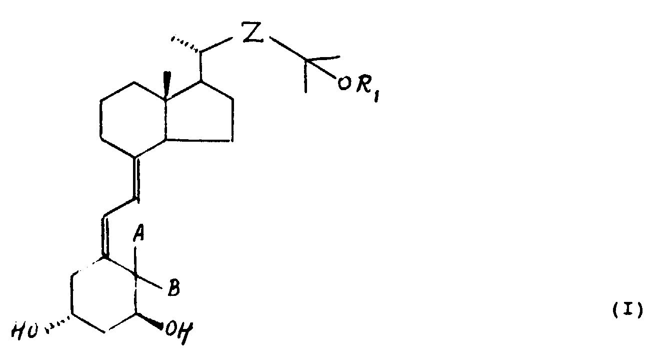 wherein:
R₁ is a hydrogen atom or a hydroxy protecting group;
Z is a group of the general formula 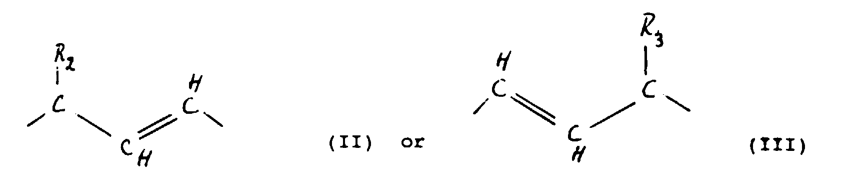 wherein
R₂ and R₃ are each individually straight or branched (C₁-C₆)alkyl groups or (C₂-C₆)alkenyl groups,
optionally substituted with one or more substituents selected from hydroxy, (C₁-C₄)alkoxy, fluoro, (C₃-C₆)cycloalkyl and (C₂-C₃)alkylenedioxy; or with unsubstituted or substituted phenyl;
or (C₃-C₆)cycloalkyl groups, or unsubstituted or substituted phenyl groups;
or wherein R₁ and R₃ together with the interconnecting oxadimethylene biradical form a 5-7 membered ring-closed (C₁-C₄)alkyl substituted hemiacetal;
with the proviso, that R₂ and R₃ are not methyl groups;
A and B are each individually hydrogen atoms or methyl groups, or
A and B form together a methylene group.

2. A vitamin D compound as claimed in Claim 1, having the general formula 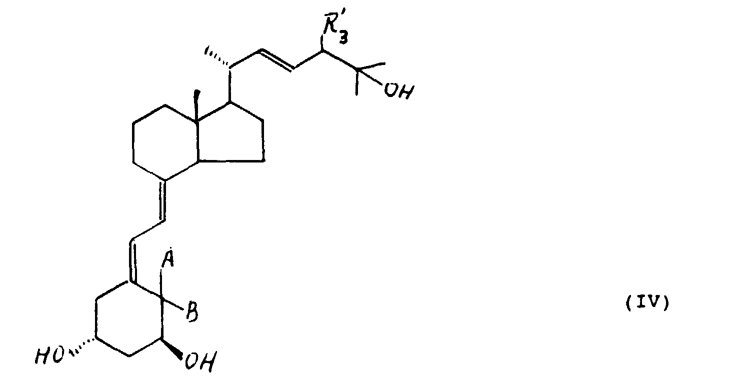 wherein:
A and B have the meanings given in Claim 1, and
R₃' is a straight or branched (C₂-C₆)alkyl group, optionally substituted with hydroxy, (C₁-C₂)alkoxy or (C₂-C₃)alkylenedioxy, a cyclopropyl group or a phenyl group.

3. A vitamin D compound as claimed in Claim 2, having the general formula IV, shown in Claim 2, wherein the symbols have the meanings given in Claim 2, on the understanding that C-24, to which substituent R₃' is attached, has either the **R** or the **S** configuration.

4. A method of preparing a vitamin D compound as claimed in Claim 1, characterized in that a hydrindane compound of the general formula 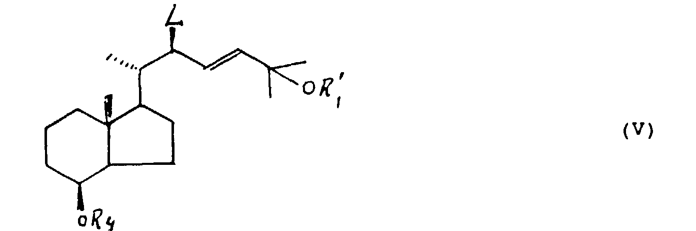 wherein:
L is a leaving group, and
R₁' and R₄ are each individually hydroxy-protecting groups; is reacted with a reagent of the general formula
R₃-MgX or R₃-Li
wherein:
R₃ has the meaning given in Claim 1, and
X is a halogen atom,
in the presence of a Cu(I) compound;
after which the hydrindane intermediate obtained, having the general formula 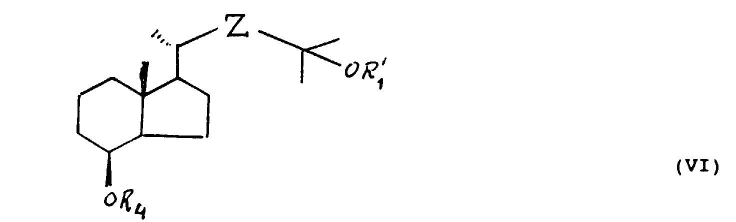 wherein Z has the meaning given in Claim 1,
is oxidized, after deprotection of OR₄, to the corresponding hydrindane-4-one compound, and is then converted, in a manner known per se for related compounds,
either (a) with a Wittig reagent of the general formula 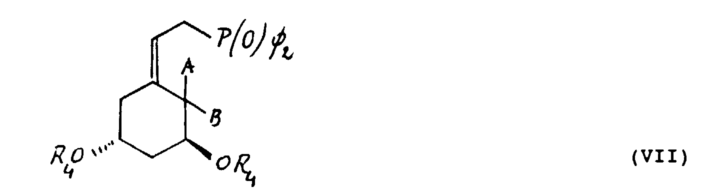 wherein:
R₄ has the above meaning, and
A and B have the meanings given in Claim 1,
or (b), after enolization, with an enyne compound of the general formula 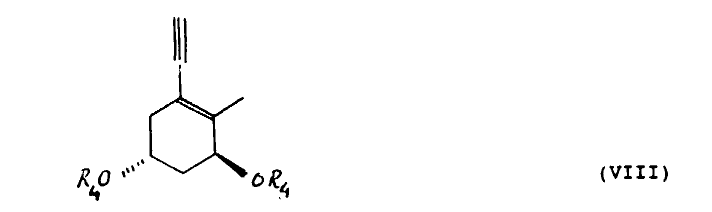 wherein R₄ has also the above meaning, followed by hydrogenation and isomerization, to produce a compound of the general formula I, wherein A and B form together a methylene group;
followed by deprotection.

5. A hydrindane intermediate which can be used in the method of Claim 4 and which has the general formula 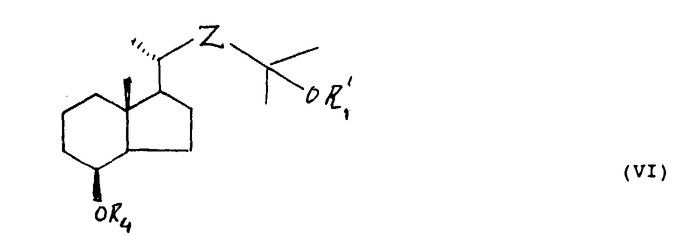 wherein:
Z has the meaning given in Claim 1, and
R₁' and R₄ have the meanings given in Claim 4.

6. A hydrindane intermediate as claimed in claim 5, having the general formula 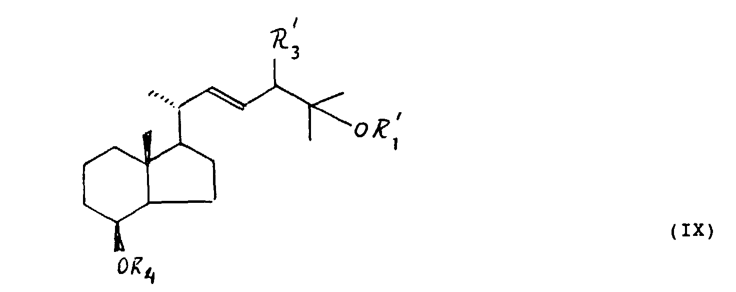 wherein:
R₃' has the meaning given in Claim 2, and
R₁' and R₄ have the meanings given in Claim 4.

7. A hydrindane intermediate as claimed in Claim 6, having the general formula IX, wherein the C-atom, to which substituent R₁' is attached, has either the **R** or the **S** configuration.

8. A method of preparing a hydrindane intermediate as claimed in Claim 5, characterized in that a compound of the general formula VI, shown in Claim 5, wherein the symbols have the meanings given in Claim 5, is prepared by reacting a hydrindane compound of the general formula 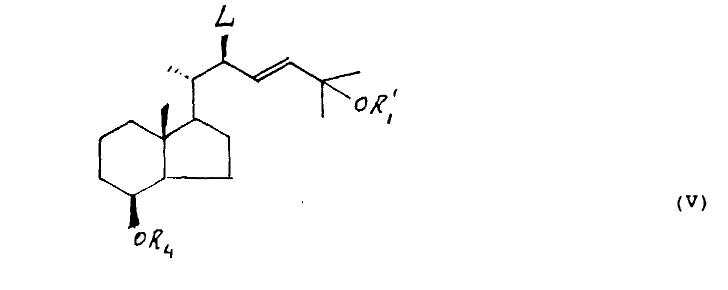 wherein L, R₁' and R₄ have the meanings given in Claim 4;
with a reagent of the general formula
R₃-MgX or R₃-Li
wherein R₃ has the meaning given in Claim 1 and X is a halogen atom, in the presence of a Cu(I) compound.

9. A method as claimed in Claim 8, characterized in that a compound of the general formula IX is prepared, wherein the C-atom, to which substituent R₃' is attached, has the configuration of formula IXA 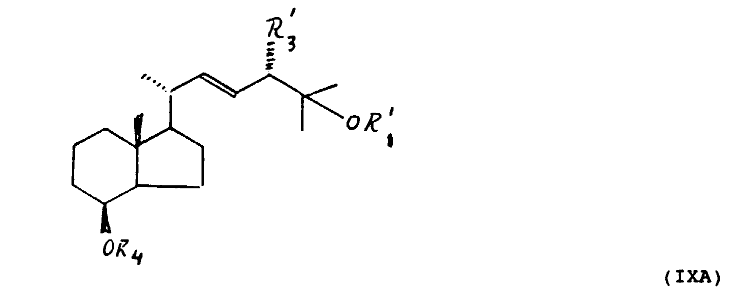 by reacting a hydrindane compound of the general formula 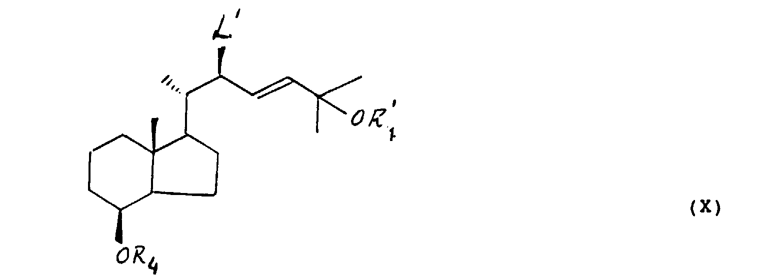 wherein:
R₁' and R₄ have the meanings given in Claim 4, and
L' is a di(hydrocarbyl)phosphate leaving group;
with a reagent of the general formula
R₃-MgX or R₃-Li
wherein R₃ has the meaning given in Claim 1 and X is a halogen atom,
in the presence of a Cu(I) compound.

10. A method as claimed in Claim 8, characterized in that a compound of the general formula IX is prepared, wherein the C-atom, to which substituent R₃' is attached, has the configuration of formula IXB 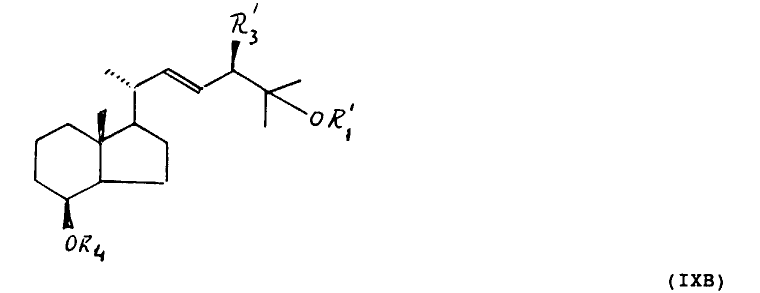 by reacting a hydrindane compound of the general formula 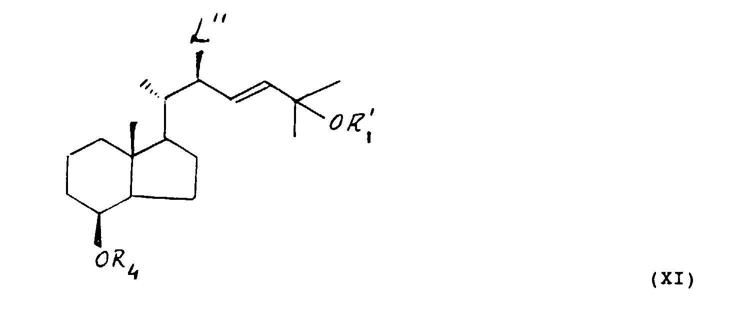 wherein:
R₁' and R₄ have the meanings given in Claim 4, and
L'' is a N-arylcarbamate leaving group;
with a reagent of the general formula
R₃-MgX or R₃-Li
wherein R₃ has the meaning given in Claim 1 and X is a halogen atom, in the presence of a Cu(I) compound.

11. A pharmaceutical composition comprising, in addition to a pharmaceutically acceptable carrier and/or at least one pharmaceutically acceptable auxiliary substance, as the active ingredient at least one compound as defined in Claim 1,2 or 3 in an effective amount.

12. A composition as claimed in claim 11 when for use in a method for treatment and/or prevention of a number of skin disorders or of a number of diseases, including bone disseases such as osteoporosis, renal osteodystrophy and osteomalacia, autoimmune diseases, acne, alopecia, skin aging, imbalance in the immune system, inflammatory diseases such as rheumatoid arthritis and asthma, as well as diseases related to abnormal cell differentiation and/or proliferation, as well as solid, skin or blood cancers, in a warm-blooded living being, comprising administering to said being or treating said being with a composition as claimed in Claim 11 in a quantity effective for the intended purpose.

## Patentansprüche

1. Vitamin D-Verbindung der allgemeinen Formel 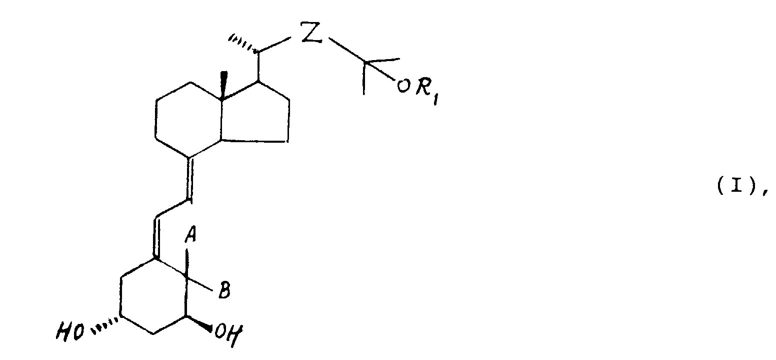 worin:
R₁ ein Wasserstoffatom oder eine Hydroxy-Schutzgruppe bedeutet;
Z eine Gruppe darstellt der allgemeinen Formel 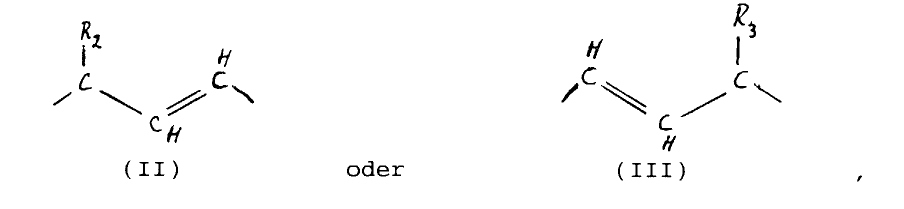
worin R₂ und R₃ jeweils einzeln bedeuten: gerade oder verzweigte (C₁-C₆)Alkyl-Gruppen oder (C₂-C₆)Alkenyl-Gruppen,
gegebenenfalls substituiert mit einem oder mehreren Substituenten, ausgewählt aus Hydroxy, (C₁-C₄)Alkoxy, Fluor, (C₃-C₆)Cycloalkyl und (C₂-C₃)Alkylendioxy; oder mit unsubstituiertem oder substituiertem Phenyl;
oder (C3-C6)Cycloalkyl-Gruppen, oder unsubstituierte oder substituierte Phenyl-Gruppen;
oder worin R₁ und R₃ zusammen mit dem sie verbindenden Oxadimethylenbirest einen 5- bis 7-gliedrigen, im Ring geschlossenen (C₁-C₄)Alkyl-substituierten Hemiacetal bilden;
mit der Maßgabe, daß R₂ und R₃ keine Methyl-Gruppen sind;
A und B jeweils einzeln Wasserstoffatome oder Methyl-Gruppen bedeuten, oder A und B zusammen eine Methylen-Gruppe bilden.

2. Vitamin D-Verbindung nach Anspruch 1 der allgemeinen Formel 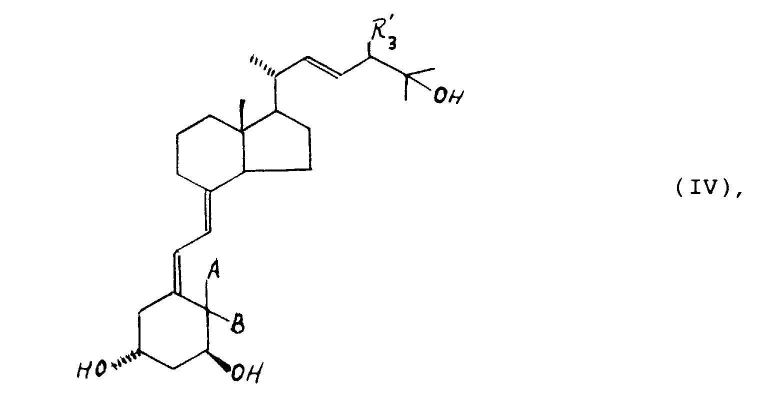 worin:
A und B die in Anspruch 1 angegebenen Bedeutungen haben, und
R₃' darstellt: eine gerade oder verzweigte (C₂-C₆)Alkyl-Gruppe, gegebenenfalls substituiert mit Hydroxy, (C₁-C₂)Alkoxy oder (C₂-C₃)Alkylendioxy, einer Cyclopropyl-Gruppe oder einer Phenyl-Gruppe.

3. Vitamin D-Verbindung nach Anspruch 2 der in Anspruch 2 gezeigten allgemeinen Formel (IV), worin die Symbole die in Anspruch 2 angegebenen Bedeutungen haben, mit der Maßgabe, daß C-24, an welchen Substituenten R₃' gebunden ist, entweder R- oder S-Konfiguration aufweist.

4. Verfahren zur Herstellung einer Vitamin D-Verbindung nach Anspruch 1, dadurch gekennzeichnet, daß eine Hydrindan-Verbindung der allgemeinen Formel 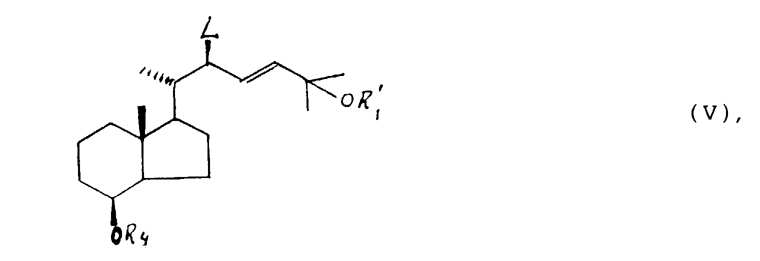 worin:
L eine Abgangsgruppe bedeutet, und
R₁' und R₄ jeweils einzeln Hydroxy-Schutzgruppen darstellen;
umgesetzt wird mit einem Reagenz der allgemeinen Formel
R₃-MgX oder R₃-Li
worin:
R₃ die in Anspruch 1 angegebene Bedeutung hat, und
X ein Halogenatom darstellt,
in Anwesenheit einer Cu(I)-Verbindung;
wonach die erhaltene Hydrindan-Zwischenverbindung der allgemeinen Formel 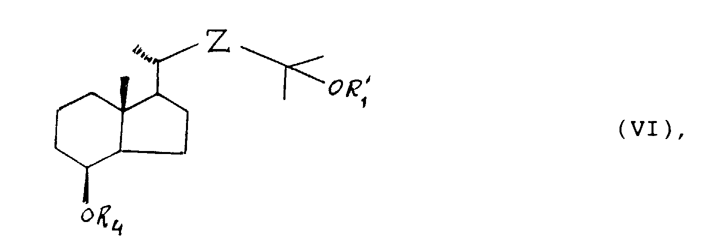 worin Z die in Anspruch 1 angegebene Bedeutung hat;
nach Entschützen von OR₄, zur entsprechenden Hydrindan-4-on-Verbindung oxidiert wird, und dann auf an sich für verwandte Verbindungen bekannte Weise übergeführt wird
entweder (a) mit einem Wittig-Reagenz der allgemeinen Formel 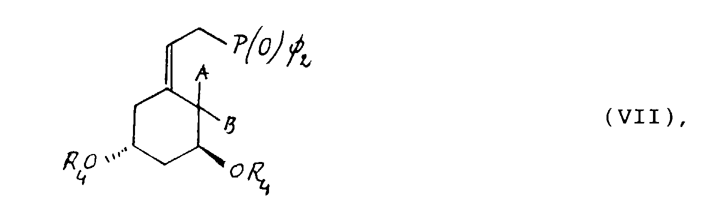 worin:
R₄ die obige Bedeutung hat, und
A und B die in Anspruch 1 angegebenen Bedeutungen haben;
oder (b), nach Enolisierung, mit einer Enyn-Verbindung der allgemeinen Formel 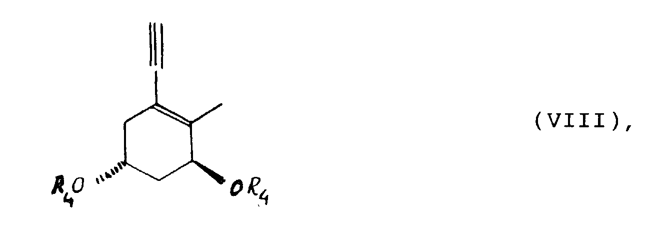 worin R₄ auch die obige Bedeutung hat, gefolgt von Hydrierung und Isomerisierung, zur Herstellung einer Verbindung der allgemeinen Formel (I), worin A und B zusammen eine Methylen-Gruppe bilden; gefolgt von Entschützen.

5. Hydrindan-Zwischenverbindung, welche im Verfahren nach Anspruch 4 verwendet werden kann, und welche die allgemeine Formel aufweist: 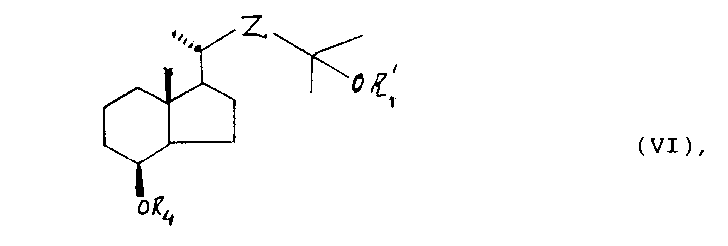 worin:
Z die in Anspruch 1 angegebene Bedeutung hat, und
R₁' und R₄ die in Anspruch 4 angegebenen Bedeutungen haben.

6. Hydrindan-Zwischenverbindung nach Anspruch 5 der allgemeinen Formel 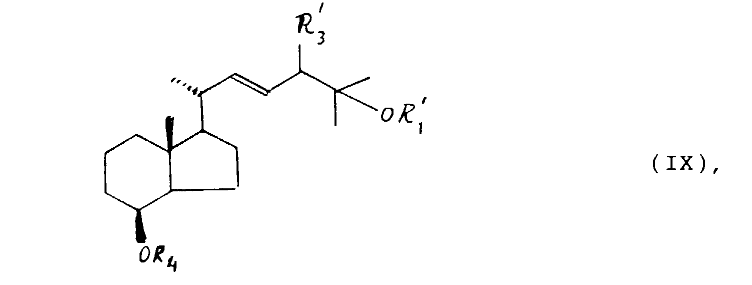 worin:
R₃' die in Anspruch 2 angegebene Bedeutung hat, und
R₁' und R₄ die in Anspruch 4 angegebenen Bedeutungen haben.

7. Hydrindan-Zwischenverbindung nach Anspruch 6 der allgemeinen Formel (IX), worin das C-Atom, an das der Substituent R₁' gebunden ist, eritweder R- oder S-Konfiguration aufweist.

8. Verfahren zur Herstellung einer Hydrindan-Zwischenverbindung nach Anspruch 5, dadurch gekennzeichnet, daß eine Verbindung der in Anspruch 5 gezeigten allgemeinen Formel (VI), worin die Symbole die in Anspruch 5 angegebenen Bedeutungen haben, hergestellt wird durch das Umsetzen einer Hydrindan-Verbindung der allgemeinen Formel 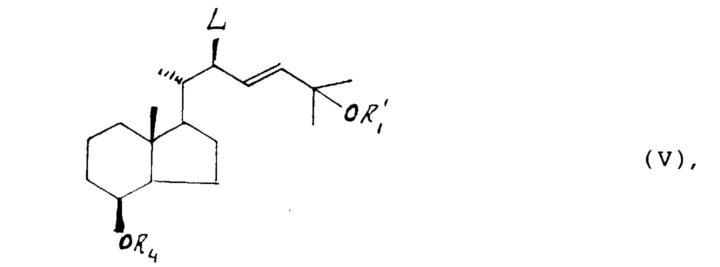 worin L, R₁' und R₄ die in Anspruch 4 angegebenen Bedeutungen haben; mit einem Reagenz der allgemeinen Formel
R₃-MgX oder R₃-Li ,
worin R₃ die in Anspruch 1 angegebene Bedeutung hat, und X ein Halogenatom darstellt, in Anwesenheit einer Cu(I)-Verbindung.

9. Verfahren nach Anspruch 8, dadurch gekennzeichnet, daß eine Verbindung der allgemeinen Formel (IX) hergestellt wird, worin das C-Atom, an das der Substituent R₃' gebunden ist, die Konfiguration der Formel (IXA) 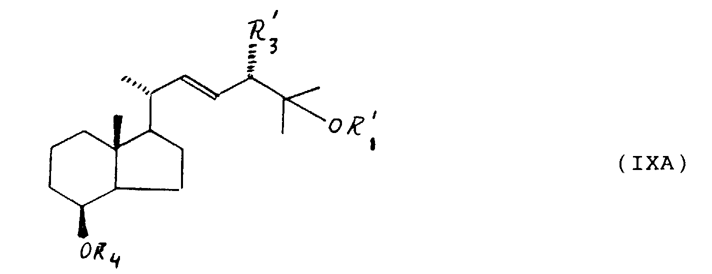 aufweist, durch das Umsetzen einer Hydrindan-Verbindung der allgemeinen Formel 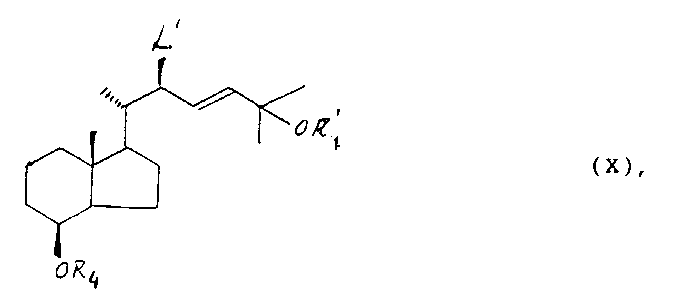 worin:
R₁' und R₄ die in Anspruch 4 angegebenen Bedeutungen haben, und
L' eine Di-(hydrocarbyl)-phosphat-Abgangsgruppe darstellt;
mit einem Reagenz der allgemeinen Formel
R₃-MgX oder R₃-Li ,
worin R₃ die in Anspruch 1 angegebene Bedeutung hat, und X ein Halogenatom darstellt, in Anwesenheit einer Cu(I)-Verbindung.

10. Verfahren nach Anspruch 8, dadurch gekennzeichnet, daß eine Verbindung der allgemeinen Formel (IX) hergestellt wird, worin das C-Atom, an das der Substituent R₃' gebunden ist, die Konfiguration der Formel (IXB) 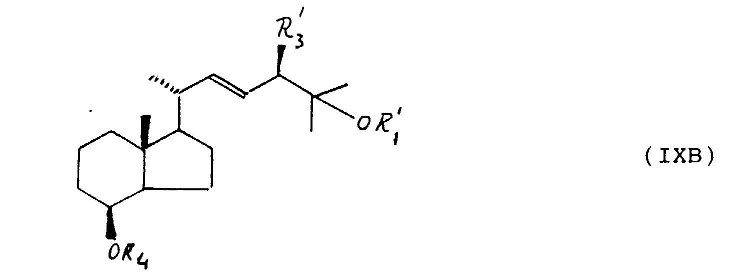 aufweist, durch das Umsetzen einer Hydrindan-Verbindung der allgemeinen Formel 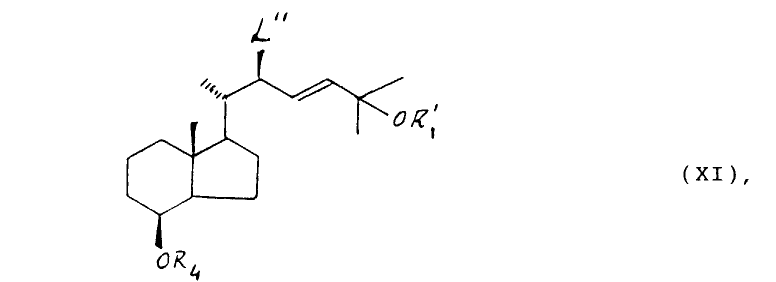 worin:
R₁' und R₄ die in Anspruch 4 angegebenen Bedeutungen haben, und
L" eine N-Arylcarbamat-Abgangsgruppe darstellt;
mit einem Reagenz der allgemeinen Formel
R₃-MgX oder R₃-Li ,
worin R₃ die in Anspruch 1 angegebene Bedeutung hat, und X ein Halogenatom darstellt, in Anwesenheit einer Cu(I)-Verbindung.

11. Pharmazeutische Zusammensetzung, welche, zusätzlich zu einem pharmazeutisch annehmbaren Träger und/oder zumindest einer pharmazeutisch annehmbaren Hilfssubstanz, als aktiven Bestandteil zumindest eine wie in Anspruch 1, 2 oder 3 definierte Verbindung in einer wirksamen Menge umfaßt.

12. Zusammensetzung nach Anspruch 11, wenn sie zur Verwendung in einem Verfahren zur Behandlung und/oder Prävention einer Reihe von Hautkrankheiten oder einer Reihe von Erkrankungen dient, einschließlich Knochenerkrankungen, wie Osteoporose, renale Osteodystrophie, Osteodystrophie und Osteomalazie, Autoimmmunkrankheiten, Akne, Alopezie, Hautalterung, Ungleichgewicht des Immunsystems, entzündliche Erkrankungen, wie rheumatoide Arthritis und Asthma, sowie Krankheiten, die mit abnormaler Zelldifferenzierung und/oder Proliferation in Zusammenhang stehen, sowie feste Tumoren, Haut- oder Blutkrebs, bei einem warmblütigen Lebewesen, umfassend die Verabreichung einer Zusammensetzung nach Anspruch 11 in einer für den beabsichtigten Zweck wirksamen Menge an das Lebewesen oder Behandeln des Lebewesens damit.

## Revendications

1. Composé de vitamine D de formule générale 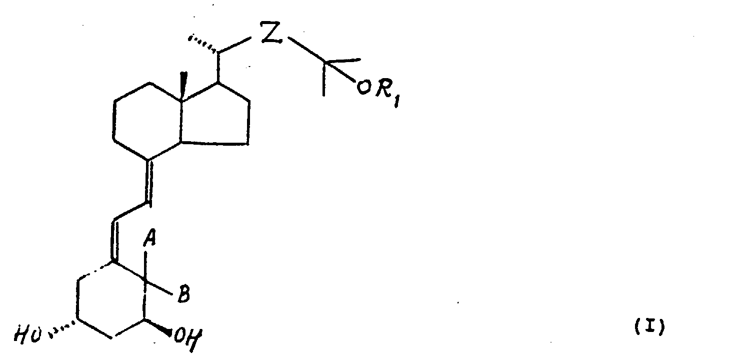 dans laquelle :
R₁ est un atome d'hydrogène ou un groupe protégeant un groupe hydroxy ;
Z est un groupe de formule générale 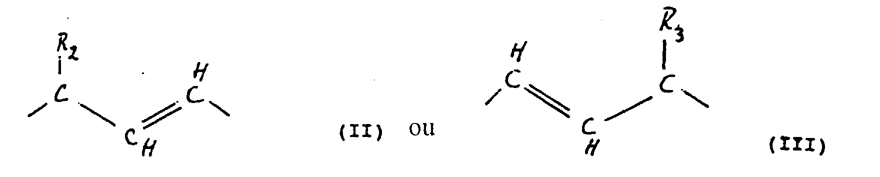
dans lesquelles R₂ et R₃ sont chacun individuellement des groupes alkyles en C₁ à C₆ ou groupes alcényles en C₂ à C₆ linéaires ou ramifiés,
éventuellement substitués par un ou plusieurs substituants choisis parmi les radicaux hydroxy, alcoxy en C₁ à C₄, fluoro, cycloalkyles en C₃ à C₆ et alkylènedioxy en C₂ à C₃ ; ou par un groupe phényle non substitué ou substitué ;
ou bien des groupes cycloalkyles en C₃ à C₆, ou des groupes phényle non substitués ou substitués ;
ou dans lesquelles R₁ et R₃, avec le biradical oxadiméthylène d'interconnexion, forment un hémiacétal substitué par un groupe alkyle en C₁ à C₄ à cycle à 5 à 7 chaînons fermé ;
à condition que R₂ et R₃ ne sont pas des groupes méthyle ;
A et B sont chacun individuellement des atomes d'hydrogène ou des groupes méthyle, ou bien A et B forment ensemble un groupe méthylène.

2. Composé de vitamine D selon la revendication 1, ayant la formule générale 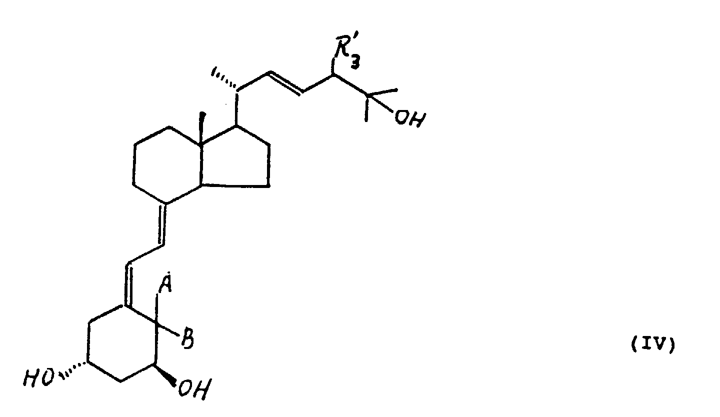 dans laquelle :
A et B ont les significations présentées dans la revendication 1, et
R₃' est un groupe alkyle en C₂ à C₆ linéaire ou ramifié, éventuellement substitué par un groupe hydroxy, alcoxy en C₁ à C₂ ou alkylènedioxy en C₂ à C₃, un groupe cyclopropyle ou un groupe phényle.

3. Composé de vitamine D selon la revendication 2, ayant la formule générale IV présentée dans la revendication 2, dans laquelle les symboles ont les significations présentées dans la revendication 2, étant entendu que C-24, auquel le substituant R₃' est attaché, a la configuration soit R soit S.

4. Procédé de préparation d'un composé de vitamine D selon la revendication 1, caractérisé en ce qu'un composé hydrindane de formule générale 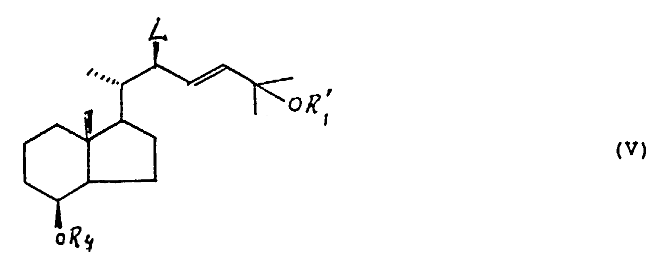 dans laquelle :
L est un groupe partant, et
R₁' et R₄ sont chacun individuellement des groupes protégeant un groupe hydroxy ;
est mis à réagir avec un réactif de formule générale
R₃-MgX ou R₃-Li
dans lesquelles :
R₃ a la signification présentée dans la revendication 1, et
X est un atome d'halogène.
en présence d'un composé de Cu(I) ;
après quoi le produit intermédiaire hydrindane obtenu, ayant la formule générale 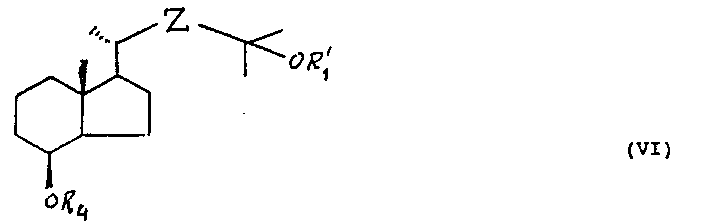 dans laquelle Z a la signification présentée dans la revendication 1,
est oxydé, après déprotection de OR₄, en le composé hydrindane-4-one correspondant, puis est converti, d'une manière connue en elle-même pour les composés apparentés,
soit (a) avec un réactif de Wittig de formule générale 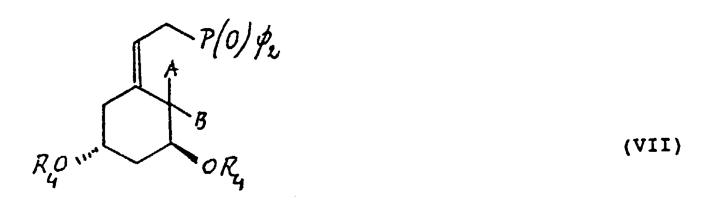 dans laquelle :
R₄ a la signification ci-dessus, et
A et B ont les significations présentées dans la revendication 1,
soit (b), après énolisation, avec un composé ényne de formule générale 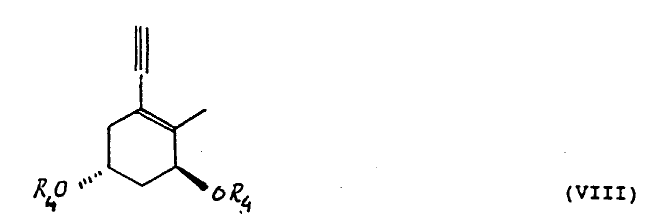 dans laquelle R₄ a aussi la signification ci-dessus, puis est hydrogéné et isomérisé, pour produire un composé de formule générale I, dans laquelle A et B forment ensemble un groupe méthylène ;
puis est déprotégé.

5. Produit intermédiaire hydrindane qui peut être utilisé dans le procédé de la revendication 4 et qui a la formule générale 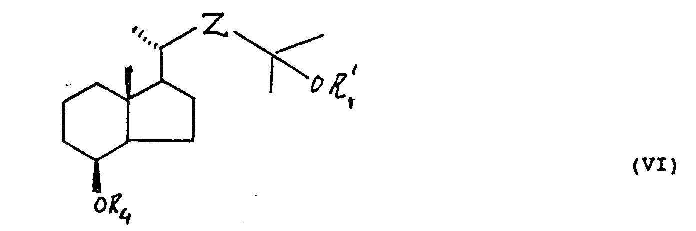 dans laquelle :
Z a la signification présentée dans la revendication 1, et
R₁' et R₄ ont les significations présentées dans la revendication 4.

6. Produit intermédiaire hydrindane selon la revendication 5, ayant la formule générale 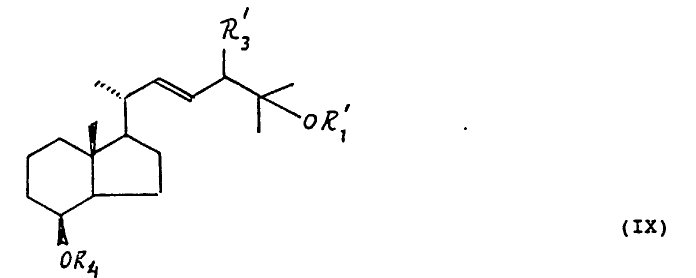 dans laquelle :
R₃' a la signification présentée dans la revendication 2, et
R₁' et R₄ ont les significations présentées dans la revendication 4.

7. Produit intermédiaire hydrindane selon la revendication 6, ayant la formule générale IX, dans laquelle l'atome de C auquel le substituant R₁' est attaché a la configuration soit R soit S.

8. Procédé de préparation d'un produit intermédiaire hydrindane selon la revendication 5, caractérisé en ce qu'un composé de formule générale VI, présentée dans la revendication 5, dans laquelle les symboles ont les significations présentées dans la revendication 5, est préparé en faisant réagir un composé hydrindane de formule générale 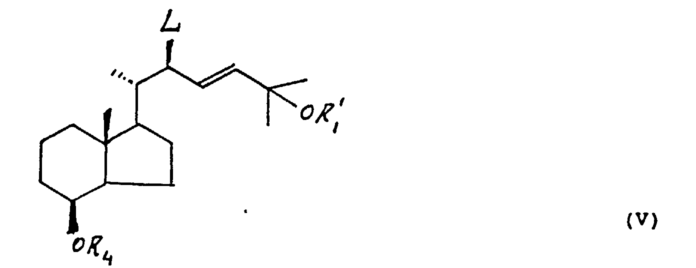 dans laquelle L, R₁' et R₄ ont les significations présentées dans la revendication 4 ;
avec un réactif de formule générale
R₃-MgX ou R₃-Li
dans lesquelles R₃ a la signification présentée dans la revendication 1, et X est un atome d'halogène, en présence d'un composé de Cu(I).

9. Procédé selon la revendication 8, caractérisé en ce qu'on prépare un composé de formule générale IX, dans laquelle l'atome de C auquel le substituant R₃' est attaché a la configuration de la formule IXA 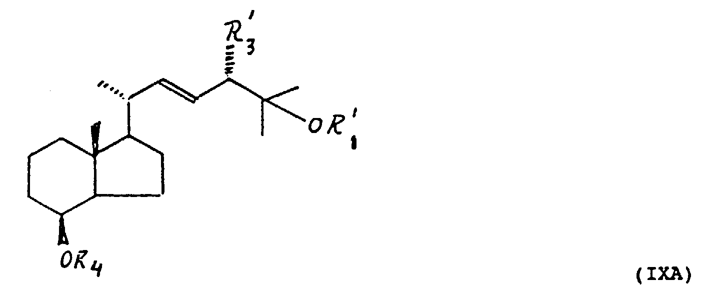 en faisant réagir un composé hydrindane de formule générale 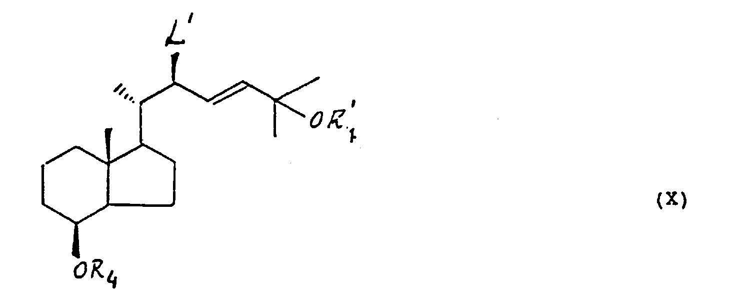 dans laquelle :
R₁' et R₄ ont les significations présentées dans la revendication 4, et
L' est un groupe partant phosphate de di(hydrocarbyle) ;
avec un réactif de formule générale
R₃-MgX ou R₃-Li
dans lesquelles R₃ a la signification présentée dans la revendication 1, et X est un atome d'halogène, en présence d'un composé de Cu(I).

10. Procédé selon la revendication 8, caractérisé en ce qu'on prépare un composé de formule générale IX, dans laquelle l'atome de C auquel le substituant R₃' est attaché a la configuration de la formule IXB 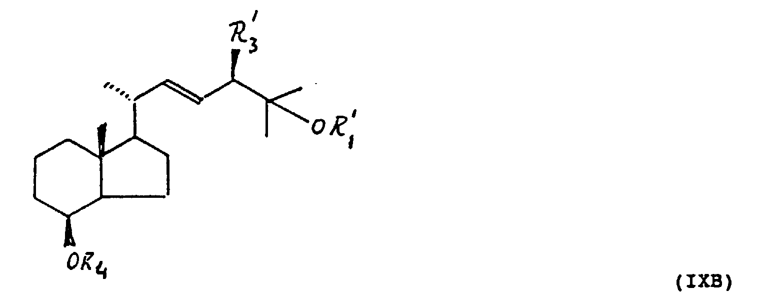 en faisant réagir un composé hydrindane de formule générale 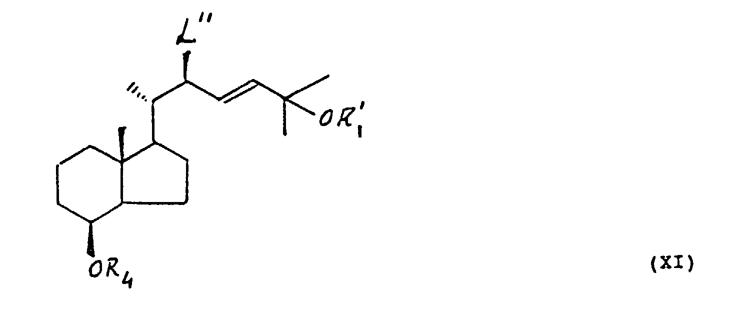 dans laquelle :
R₁' et R₄ ont les significations présentées dans la revendication 4, et
L" est un groupe partant carbamate de N-aryle ;
avec un réactif de formule générale
R₃-MgX ou R₃-Li
dans lesquelles R₃ a la signification présentée dans la revendication 1, et X est un atome d'halogène, en présence d'un composé de Cu(I).

11. Composition pharmaceutique, comprenant, en plus d'un véhicule pharmaceutiquement acceptable et/ou d'au moins une substance auxiliaire pharmaceutiquement acceptable, en tant que principe actif, au moins un composé tel que défini dans la revendication 1, 2 ou 3, en une quantité efficace.

12. Composition selon la revendication 11, lorsqu'elle est utilisée dans un procédé pour le traitement et/ou la prévention d'un certain nombre de maladies de la peau ou d'un certain nombre de maladies, incluant des maladies osseuses telles que l'ostéoporose, l'ostéodystrophie rénale et l'ostéomalacie, des maladies auto-immunes, l'acné, l'alopécie, le vieillissement de la peau, un déséquilibre du système immunitaire, des maladies inflammatoires telles que la polyarthrite rhumatoïde et l'asthme, ainsi que des maladies liées à une différentiation et/ou prolifération cellulaires anormales, ainsi que des cancers solides, de la peau ou du sang, chez un être vivant à sang chaud, comprenant l'administration audit être ou le traitement dudit être avec une composition selon la revendication 11 en une quantité efficace pour l'objectif recherché.
